# EUROPEAN PATENT SPECIFICATION

(11) **EP 4 309 660 B1**
(45) Date of publication and mention of the grant of the patent: **15.07.2026**
(21) Application number: 22185583.6
(22) Date of filing: 18.07.2022
(51) Int. Cl.: A61K 35/15, A61K 9/00, A61K 35/30, A61P 35/00, C12N 5/00

(54) **EXTRACELLULAR VESICLES DERIVED FROM MACROPHAGES AND MICROGLIA FOR USE IN TREATING CANCER**
VON MAKROPHAGEN UND MIKROGLIA ABGELEITETE EXTRAZELLULÄRE VESIKEL ZUR BEHANDLUNG VON KREBS
VÉSICULES EXTRACELLULAIRES DÉRIVÉES DE MACROPHAGES ET DE MICROGLIES POUR LE TRAITEMENT DU CANCER

(43) Date of publication of application: 24.01.2024
(73) Proprietor: Lietuvos sveikatos mokslu universitetas, 44307 Kaunas (LT)
(72) Inventor: Jekabsone, Aiste, Kaunas (LT); Kulakauskienè, Deimante, Kaunas (LT); Rudaityte, Akvile, Kaunas (LT); Balion, Zbigniev, Kaunas (LT); Masaityte, Aiste, Kaunas (LT); Pauziene, Neringa, Kaunas (LT); Skredeniene, Ruta, Kaunas (LT); Jukneviciene, Milda, Kaunas (LT); Balnyte, Ingrida, Kaunas (LT); Sadauskiene, Ilona, Kaunas (LT); Mikniene, Zoja, Kaunas (LT); Vilkickyte, Gabriele, Kaunas (LT); Raudone, Lina, Kaunas (LT); Sventoraitiene, Jurgita, Kaunas (LT); Inokaitis, Hermanas, Kaunas (LT)
(74) Representative: AAA Law

(56) References cited:
- WO-A1-2017/173034
- WO-A1-2020/032379
- WO-A1-2022/006178
- WO-A2-2019/133934
- FU SHENGYANG ET AL: "Exosome engineering: Current progress in cargo loading and targeted delivery", NANOIMPACT, vol. 20, 1 October 2020 (2020-10-01), pages 100261, XP055957415, ISSN: 2452-0748, DOI: 10.1016/j.impact.2020.100261

## Description

### FIELD OF INVENTION

The invention relates to the field of methods for deriving compositions of extracellular vesicles and particles for providing anticancer activity.

### BACKGROUND

Over 80% of malignant brain tumours consist of gliomas, of which glioblastomas (GBMs) have the highest invasiveness and the worst prognosis^{1,2}. To date, no effective treatment strategies for GBM have been developed, and surgical treatment combined with chemical, immune, and radiation therapy only delays but does not arrest the recurrence of these tumors³. The aggressiveness of GBM is due to the chemotherapy- and the radiotherapy-resistant cell subpopulation⁴ and the ability of GBM cells to rapidly migrate into tissues reducing the effectiveness of surgical and localised chemo/radiotherapy⁵. Therefore, new strategies capable of recognising and destroying both stem glioma cells and single GBM cells distant from the mother tumour need to be developed.

All cells produce extracellular vesicles (EVs) and extracellular particles (EPs) that together range from about 20 nm to above 10 µm in diameter⁶. EVs are characterised by a lipid bilayer that covers a cargo of multiple biomolecules⁷. In their turn, EPs have no lipid membrane but share some structural and functional components with EVS⁸. According to their size, EVs are classified as small (up to 1 µm) and large EVs (above 1 µm). EPs are smaller than EVs; their size is below 50 nm⁸. EVs and EPs are diverse according to their origin; they can be derived from the intracellular organoids (exomeres, supermeres, exosomes), formed from the plasma membrane (ectosomes, apoptotic bodies, and oncosomes), or shed by fast-moving cells as motility-related organoids (migrasomes)⁹⁻¹¹. There is some correlation between the particle or vesicle size and their origin; exomeres, supermeres, and exosomes belong to the small particle and vesicle group, and ectosomes, apoptotic bodies, and oncosomes are large. However, the size of different EVs and EPs is not so precisely defined and overlaps between the classes; for example, some ectosomes can be much smaller than 1 mm. EVs and EPs extend cell functions space through the targeted delivery of specific proteins, lipids, and nucleic acids, representing the status of the producer cells that different stimuli can regulate. Currently, the most investigated group is small EVs and significantly less is known about the structure and functions of large EVs and EPs.

EVs secreted by macrophages and microglia can be recognised and internalised by cancer cells, including glioblastoma^{12,23}, and can be used as efficient drug carriers to cancer cells reducing unspecific toxicity¹⁴. Moreover, the EVs from various cells are proven to cross the blood-brain barrier within an hour after intranasal delivery making them a tool for overcoming dose insufficiency in the brain¹⁵⁻¹⁷. In addition, EVs from M1 macrophages were recently approved efficient as vehicles to deliver synergistic immunomodulation, chemi-excited photodynamic, and hypoxia-activated chemotherapy in GBM *in vitro* and *in vivo* models¹⁸. Also, inflammatory-stimulated macrophage EVs were applied as a simultaneous treatment for immunomodulation of tumour environment towards anticancer activity¹⁹⁻²⁰. M1 macrophages from healthy individuals are known to exhibit tumour-suppressing activity²¹, and their EVs potentiate cancer treatment and inhibit cancer cells directly²².

Previous inventions of EV applications for cancer treatment focus on exploiting the EVs as delivery vehicles for anticancer agents (W02017173034/US20200297631)²³ or immunomodulating compounds (W02019133934/ EP3731849/US10723782)²⁴. The main difference between the current disclosure from the prior art lies in obtaining EV and EP composition for providing anticancer properties even without delivery of anticancer or another agent.

The most relevant subject matter to the present invention is disclosed in the international patent application W02017173034. It describes a composition for delivery of a biological agent to a cell comprising an exosome isolated from an immune cell, including a macrophage/monocyte. The similarities with the current disclosure are in (i) the composition that contains the exosome, which is an extracellular vesicle, (ii) the origin of the exosome which is an immune cell, including a monocyte/macrophage, (iii) the loading of the exosomes with the anticancer agent which can be doxorubicin, (iv) the use of the composition for cancer treatment, (v) the use of the composition for the cancer treatment by intravenous or intranasal delivery, and (vi) the use of the composition for treatment when the composition is made from the autologous cells of the patient.

The composition disclosed here differs from the above-indicated one in several aspects. First, it comprises not only exosomes but also other EVs and EPs. Next to the markers of exosomes, the composition is also characterised by the markers of exomeres, apoptotic bodies and migrasomes, contains mitochondrial proteins and exposed phosphatidylserine. In addition, the composition is obtained by the method of stimulation of immune cells for inflammatory response by a Toll-like receptor agonist and for migration by specific substrate stiffness and specific density of adhesion peptide RGD. The Toll-like receptor stimulation stimulates the formation of large EVs comprising inflammatory phenotype markers. Substrate stiffness and adhesion peptide-induced migration stimulate the production of large migration-related EVs. Next, the EV producer is also a microglial cell which is not mentioned in WO2017173034. Another difference is the anticancer activity of the EV composition itself instead of an anticancer drug delivery vehicle, as claimed in WO2017173034. Additionally, a method of anticancer drug loading is different from the disclosed in WO2017173034 (Claim 58: incubating the biological agent with the exosome in the presence of saponins subjecting to a freeze-thaw cycle, sonication, or extrusion); the EVs with anticancer substances in this disclosure are produced by adding them in the incubation medium of the cells. The improvement of the technology by introducing the differences is manifested by (i) a synergistic cancer cell suppressing activity of the different EV and EP populations, (ii) higher yield of the EVs with the activity because of the additional to exosomes EV populations, and (iii) more stable drug-loaded EVs because the EV membranes are not physically or chemically disrupted for drug entrance, but the drug is packed by intrinsic cellular pathways instead.

In WO2019133934, EV composition for immune-oncology and anti-inflammatory therapy is described. The inventors claim an EV comprising a cell membrane bounding an enclosed volume, wherein the EV comprises a fusion protein comprising a prostaglandin F2 receptor negative regulator or a fragment thereof fused to an immunomodulating component. The similarities are (i) the use of EV derived from a cell, (ii) the use of the EV for delivery of a bioactive compound, (iii) the use of the EV with the compound for cancer treatment, (iv) the use of the EV which is an exosome or a nanovesicle.

The differences from WO2019133934 are (i) the object of claim, which is not a composition comprised of an EV modified by a fusion protein and an immunomodulating component but a composition of multiple EVs and EPs obtained by a specific process; (ii) the EVs of the composition which are not only exosomes and nanovesicles, but also large EVs, apoptotic bodies and migrasomes; (iii) the presence of EPs with the markers of exomeres in the composition; (iv) the anticancer properties of the composition itself regardless of the carrying anticancer agent; (v) the use of the composition for providing suppressing activity on cancer cells instead of instructing immune cells of cancer microenvironment; (vi) if the composition is used for delivery of anticancer agent, the agent is not chemically attached to the EV (or EP) of the composition.

### SUMMARY

The invention is set out in the appended set of claims. In particular, a method for producing extracellular vesicles and/or extracellular particles from macrophage or microglial immune cells is claimed, wherein the method comprises:
a. stimulating the immune cells by TLR-3 agonist poly(I:C),
b. stimulating the immune cells by surface-attached cell adhesion peptide RGD,
c. stimulating the cultured immune cells by a matrix stiffness, wherein the matrix stiffness is between 8 and 1000 kPa,
d. isolating the extracellular vesicles and/or particles released to the medium of the cultured immune cells using centrifugation and/or size-exclusion chromatography, and/or polymer precipitation, and/or acoustic trapping, and/or tangential filtration,
e. detaching of the matrix-attached extracellular vesicles with Trypsin-EDTA solution and centrifugation, and/or size-exclusion chromatography, and/or polymer precipitation, and/or acoustic trapping, or tangential filtration,
f. combining extracellular vesicles and/or particles obtained in the steps 1d and 1e,
g. decreasing the size of the large extracellular vesicles in the composition by sonication or extrusion to the range of 10 to 250 nm,
h. confirming the presence of extracellular vesicles exposing phosphatidylserine,
i. confirming the presence of at least three of the proteins selected from the group of CD9, CD63, CD81, Syntenin-1, TSG-101, Alix, Hsp70, Hsp90,
j. confirming the presence of at least two of the proteins selected from the group of MAT1A, IDH1, GMPPB, AGO1, APP,
k. confirming the presence of the protein NDST1,
l. confirming the presence of COXIV and/or VDAC and/or other mitochondrial proteins,
m. confirming the presence of at least three of the proteins selected from the group of CD80, CD86, MHC-II, CD64, CD32, HLA-DR, CD11c.

Also, the invention provides a composition for use in the treatment of cancer of human or animal patients, comprising extracellular vesicles obtained by the method of invention.

EVs and EPs are natural cell-to-cell communication tools that can be exploited for cell instruction therapies. The technical problem of EV application for cancer treatment is (i) insufficient yield of EVs with desired properties, (ii) insufficient EV targeting, (iii) insufficient EV penetration, (iv) insufficient EV targeting; (v) under exploration and underuse of EPs and large EVs for providing anticancer activity, (vi) poor stability of EVs after drug loading.

The current disclosure describes methods for producing compositions of EVs and EPs derived from immune cells stimulated by Toll-like receptor agonist, microenvironment stiffness and adhesion peptides.

In the method of invention, the EVs and EPs are from macrophages or microglial cells activated by TLR-3 agonist poly(I:C), and the macrophages or microglial cells are stimulated to migrate and shed migrasomes by changing matrix stiffness and cell adhesion peptide density.

In some aspects, the composition comprises internal compartments, including mitochondrial compartments.

In some aspects, the EVs are large EVs exposing phosphatidylserine.

In some aspects, the EVs and EPs are exosomes and/or exomeres.

In some aspects the EVs and EPs contain markers of inflammatory M1 phenotype of macrophages or microglia.

In some aspects, different EV and EP fractions possess anticancer activity which is directly dependent on the time of treatment of macrophages or microglia with the TLR agonist. The anticancer activity is related to the conversion of the EV and EP-producing cell to the M1 phenotype and is increased with the addition of each EV fraction: small and medium EPs and EVs, large EVs and migrasomes. The synergistic activity of the EV fractions is shown in the examples.

In some aspects, the method comprises the treatment of the microglial and/or macrophage cells with anticancer substances to promote stable EVs with the encapsulated compounds.

In some aspects, endogenous/autogenic (coming from the subject itself) or exogenous/allogenic (coming from another subject) can be applied.

The advantageous effects of the current invention are (i) exploitation of the natural capacity of EVs and EPs from inflammatory-activated macrophages and microglial cells to recognise and eliminate cancer cells, (ii) a process that provides EVs and EPs with anticancer activity, (iii) increasing yield of the EVs and EPs with the activity (iv) allowing to use large EVs, (v) allowing to use highly penetrating, fast-moving EVs migrasomes, (vi) allowing to produce stable EV and EP compositions with anticancer agents, (vii) allowing to easily combine natural and externally added anticancer activities in the same composition.

### DESCRIPTION OF THE DRAWINGS

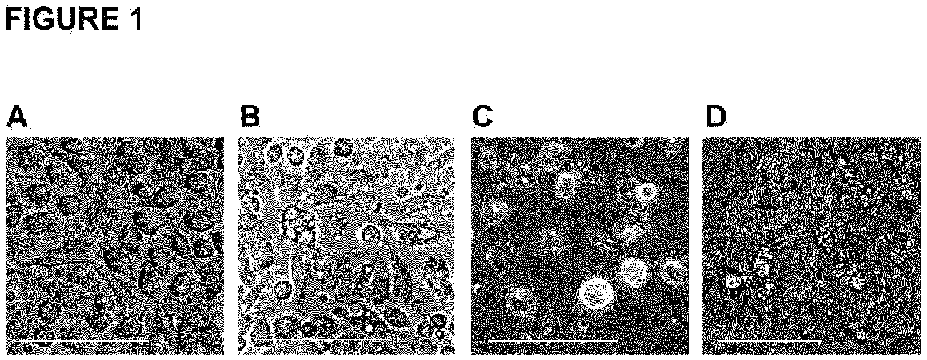

FIG. 1A shows microscopic images of U937 human monocytes converted to macrophages and shedding large EVs after 1h treatment with poly(I:C). FIG. 1B shows macrophage membrane blebbing after 8h treatment with poly(I:C). FIG. 1C shows microscopic images of HMC-3 human microglia shedding large EVs after 1h treatment with poly(I:C). FIG. 1D shows microglial membrane blebbing after 8h treatment with poly(I:C). The scale bar is 50 mm.

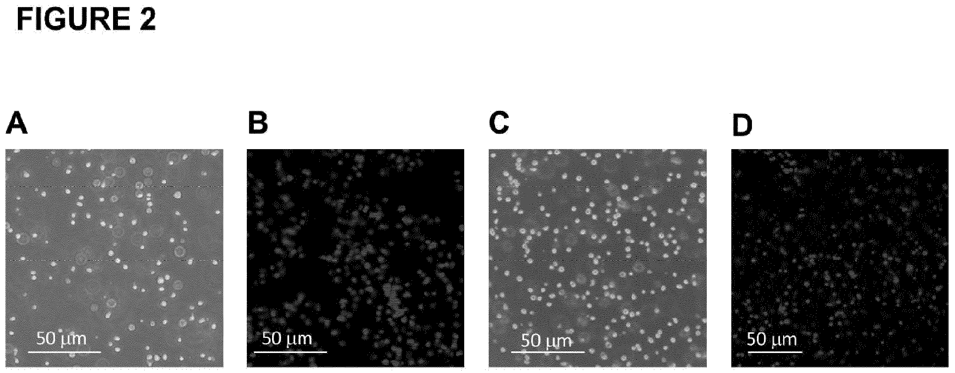

FIG. 2 shows microscopic images of large EVs in the medium collected 8 hours after poly(I:C) treatment. In FIG. 2A and FIG. 2B, the EVs are derived from macrophages in FIG. 2C and FIG. 2D - from microglia. In FIG. 2C and FIG. 2D, the vesicles are stained for phosphatidylserine exposure with Annexin V.

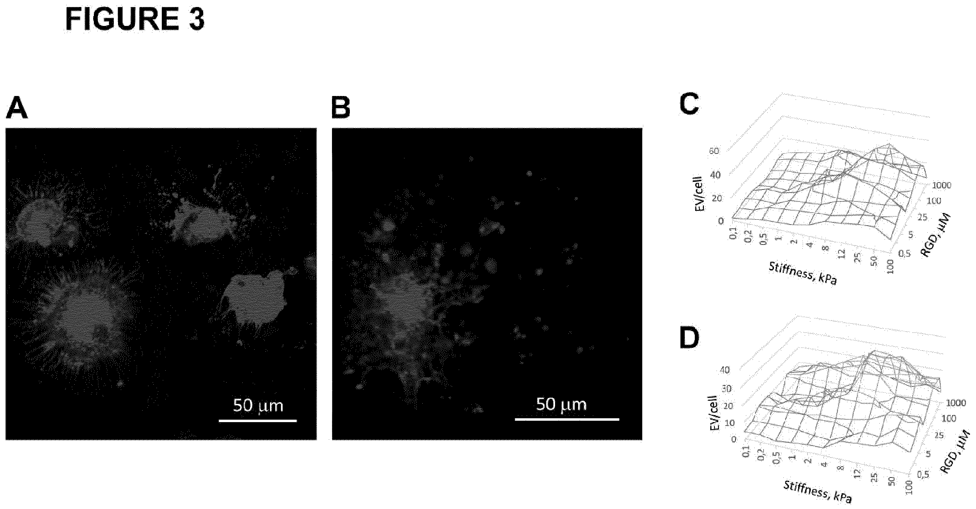

FIG. 3A shows U937-derived macrophages on RGD-coated hydrogel shedding EVs. FIG. 3B shows HMC-3 on RGD-coated hydrogel shedding EVs. FIG. 3C shows the effect of substrate stiffness and on the number of EV formation by moving U937-derived macrophages and HMC-3 microglia.

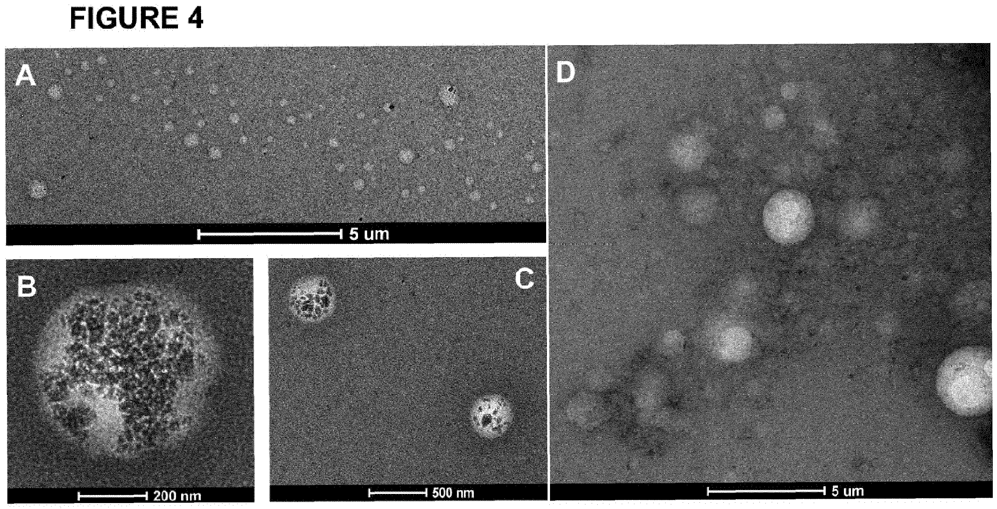

FIG. 4A shows a transmission electron microscopy image of large EVs derived from macrophages. FIG. 4B shows a transmission electron microscopy image of a macrophage-derived apoptotic body. FIG. 4C shows a transmission electron microscopy image of microglia-derived large EVs. FIG. 4D shows a transmission electron microscopy image of large and small EVs derived from microglia.

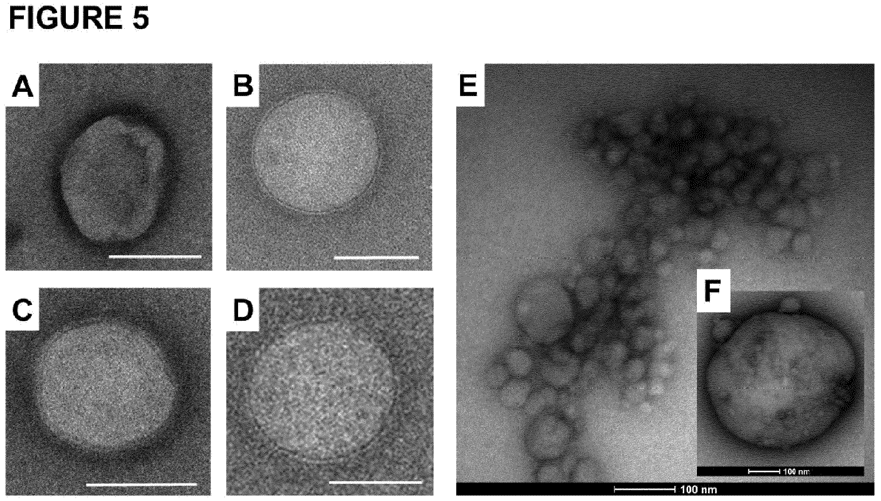

FIG. 5A shows a transmission electron microscopy image of a small macrophage-derived EV with inner volume. FIG. 5B shows a small macrophage-derived EV that has internal volume surrounded by a lipid bilayer membrane. FIG. 5C and FIG. 5D shows small microglia-derived EVs with inner volume surrounded by a lipid bilayer membrane. FIG. 5E shows a transmission electron microscopy image of a group of small macrophage-derived EVs containing EVs of size characteristic to exosomes and particles of size characteristic to exomeres. FIG. 5F shows a transmission electron microscopy image of a medium-size microglia-derived EV together with small EVs and particles of size characteristic to exomeres.

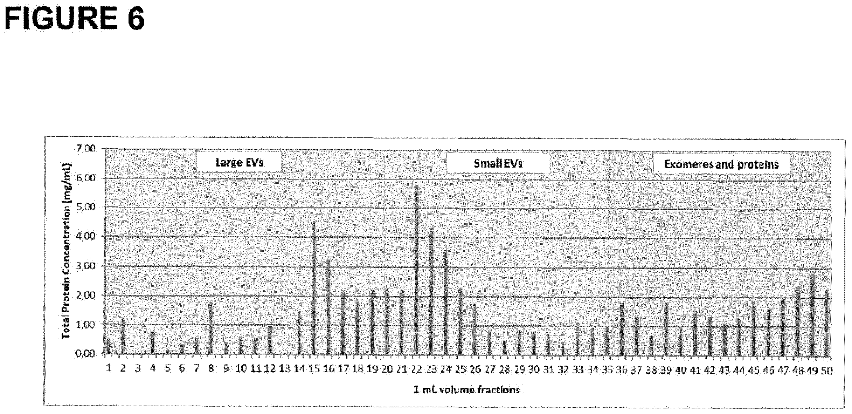

FIG. 6 shows an example of the graph plotting the presence of protein in size exclusion chromatography fractions of an EV sample containing large and small EVs and extracellular particles.

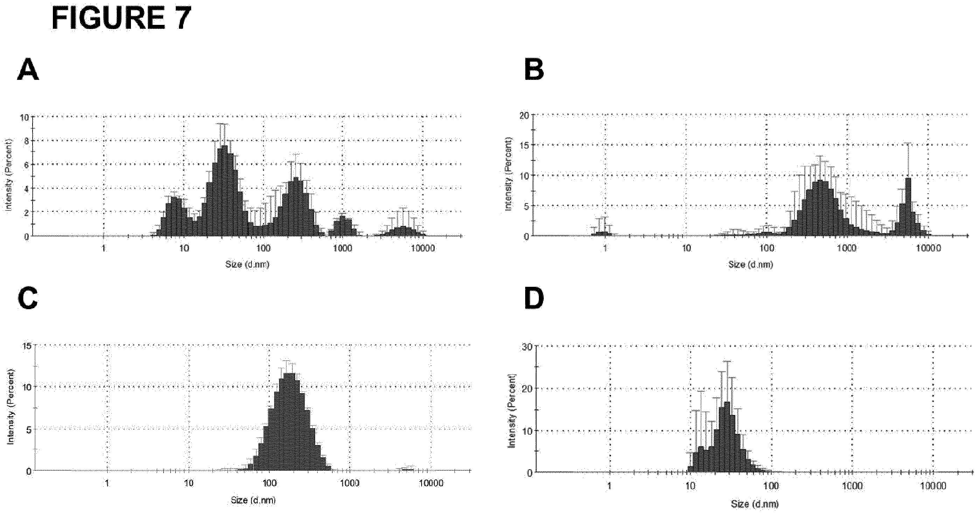

FIG. 7A shows an example of particle distribution by size in a sample of all collected EVs assessed by a Zetasizer. FIG. 7B shows an example of particle distribution by size in an isolated large EV fraction assessed by a Zetasizer. FIG. 7C shows an example of particle distribution by size in 20 - 35 post column EV fraction pool assessed by a Zetasizer. FIG. 7D shows an example of particle distribution by size in 36 - 50 EV fraction pool assessed by a Zetasizer.

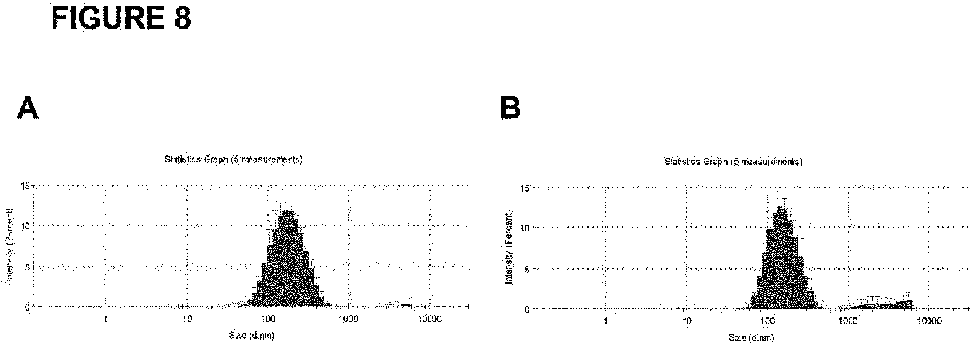

FIG. 8A shows an example of particle distribution by size in homogenised samples from U937 macrophage-derived large EVs, migrasomes and other EVs and extracellular particles assessed by a Zetasizer. FIG. 8B shows an example of particle distribution by size in homogenised samples from HMC-3 microglia-derived large EVs, migrasomes and other EVs and extracellular particles assessed by a Zetasizer.

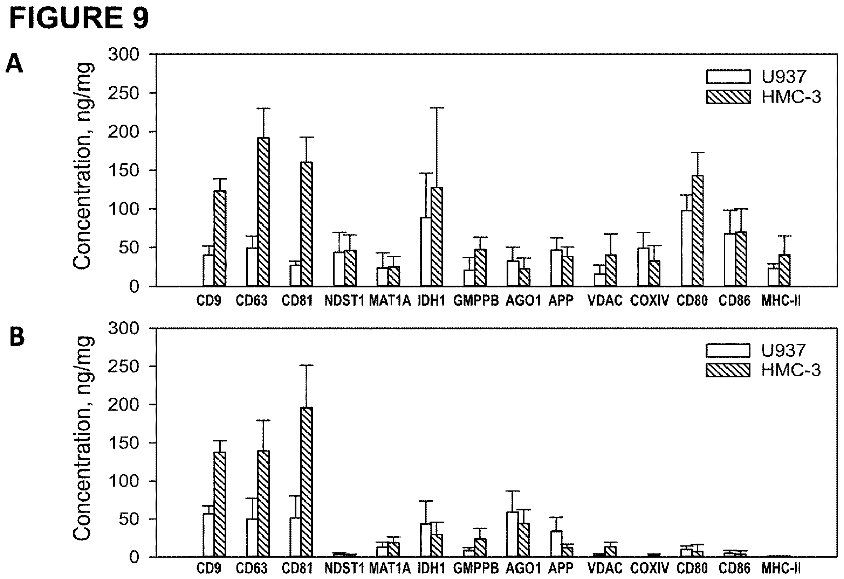

FIG. 9A shows the amount of CD9, CF63, CD81, NDST1, MAT1A, IDH1, GMPPB, AGOl, APP, VDAC, COXIV, CD80, CD86, and MHC-II proteins in EV samples derived from 12kPa RGD-coated surface and 8h poly(C:I)-treated macrophages U937 and microglia HMC-3 cells. FIG. 9B shows the amount of CD9, CF63, CD81, NDST1, MAT1A, IDH1, GMPPB, AGO1, APP, VDAC, COXIV, CD80, CD86, and MHC-II proteins in EV samples derived from untreated macrophages U937 and microglia HMC-3 cells.

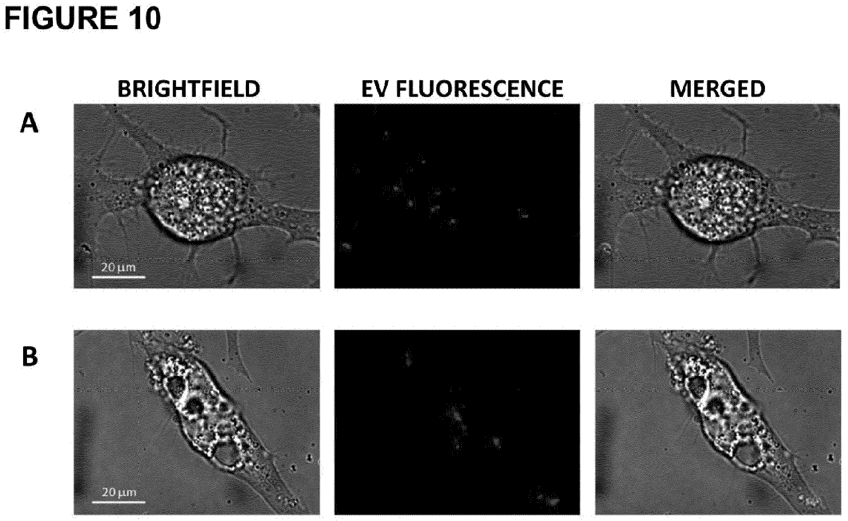

FIG. 10A shows microscopic brightfield and fluorescent images of macrophage-derived EVs with fluorescent cargo internalised by human glioblastoma HROG36 cells for 2h time after the administration in culture. FIG. 10B shows microscopic brightfield and fluorescent images of microglia-derived EVs with fluorescent cargo internalised by human glioblastoma HROG36 cells for 2h time after the administration in culture.

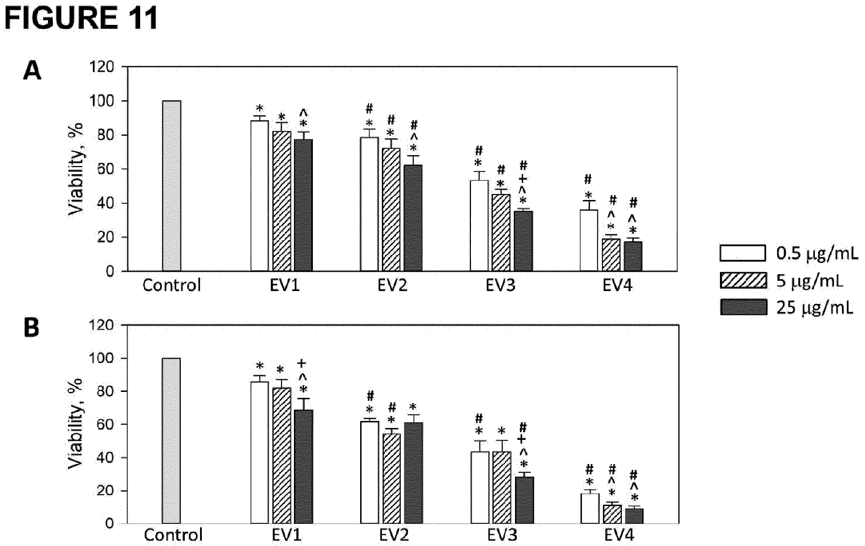

FIG. 11A shows the effect of differently concentrated EVs from untreated macrophages, EVs from 1h poly(I:C)-treated macrophages, EVs from macrophages migrating on soft RGD-coated substrate and 8h poly(I:C) macrophages (EV1, EV2, EV3 and EV4, respectively) on the viability of glioblastoma cells grown in 2D cultures. FIG. 11B shows the effect of differently concentrated EVs from untreated microglia, EVs from 1h poly(I:C) microglia. EVs from microglia migrating on the soft substrate and 8h poly(I:C)-treated microglia (EV1, EV2, EV3 and EV4, respectively) on the viability of glioblastoma cells grown in 2D cultures. The symbol * is for a statistically significant decrease compared with the Control ^ - with the 0.5 µg/mL within each EV group, + with the 5 µg/mL within each EV group, and # - with the same concentrated EV treatment in the previous group.

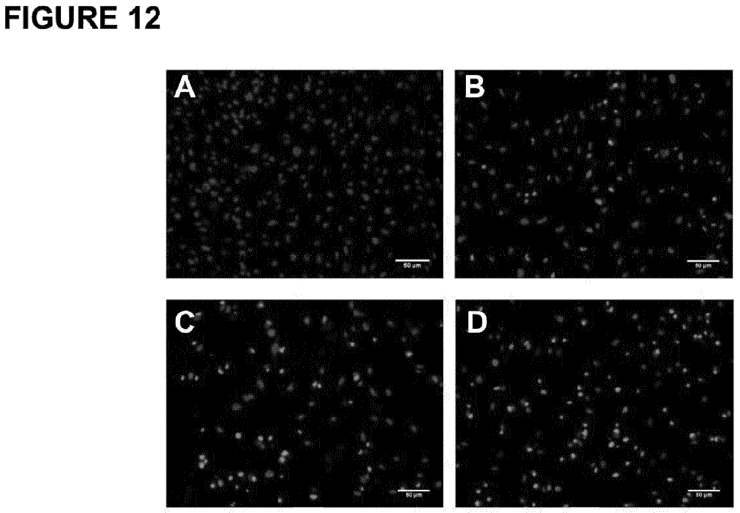

FIG. 12 shows fluorescent microscopy images of viable (blue, dark) and non-viable (red, light) nuclei of cultured glioblastoma cells. FIG. 12A shows a fluorescent microscopy image of untreated glioblastoma cells. FIG. 12B shows a fluorescent microscopy image of glioblastoma cells treated for 72 hours with 5 µg/mL migration-induced EVs on a soft RGD-coated substrate derived from U-937-derived macrophages. FIG. 12C shows a fluorescent microscopy image of glioblastoma cells treated for 72 hours with 5 µg/mL large EVs derived from 8h poly(I:C)-stimulated macrophages. FIG. 12D shows a fluorescent microscopy image of glioblastoma cells treated for 72 hours with 5 µg/mL of all range EVs derived from U937-derived macrophages (EV4 in FIG. 11A).

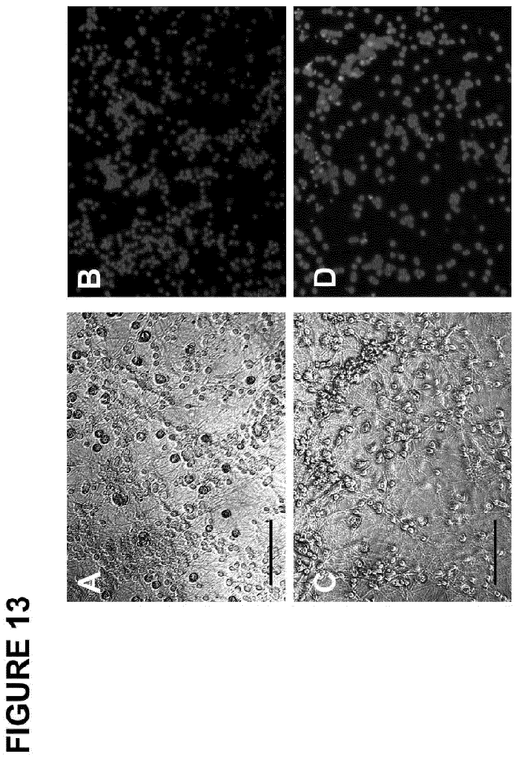

FIG. 13A shows a brightfield microscopy image of the primary mixed cerebellar neuronal-glial cells comprised of neurons, astrocytes and microglia. The scale bar is 100 mm. FIG. 13B shows the same as in FIG. 13A microscopy field image under a fluorescent microscope revealing double nuclear viability staining (viable blue, dark and non-viable red, light, respectively). The scale bar is the same as in FIG. 13A. FIG. 13C shows a brightfield microscopy image of the primary mixed cerebellar neuronal-glial cells comprised of neurons, astrocytes, and microglia 72 hours treated with EVs derived from 5 µg/mL migration-induced EVs on soft RGD-coated substrate and 8h poly(I:C)-treated macrophages (EV4 in FIG.11). The scale bar is 100 mm, FIG. 13D shows the same as in FIG. 13C microscopy field image under a fluorescent microscope revealing double nuclear viability staining (viable blue, dark and necrotic red, respectively). The scale bar is the same as in FIG. 13C.

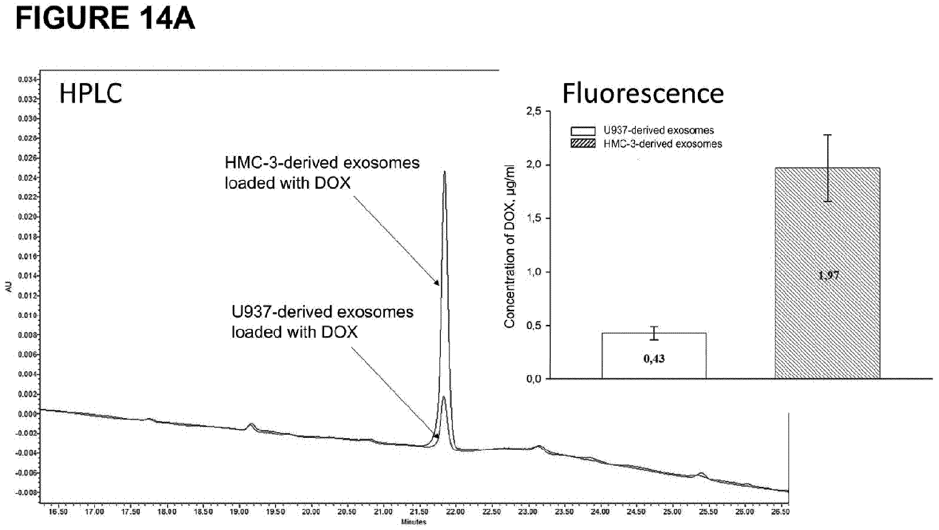

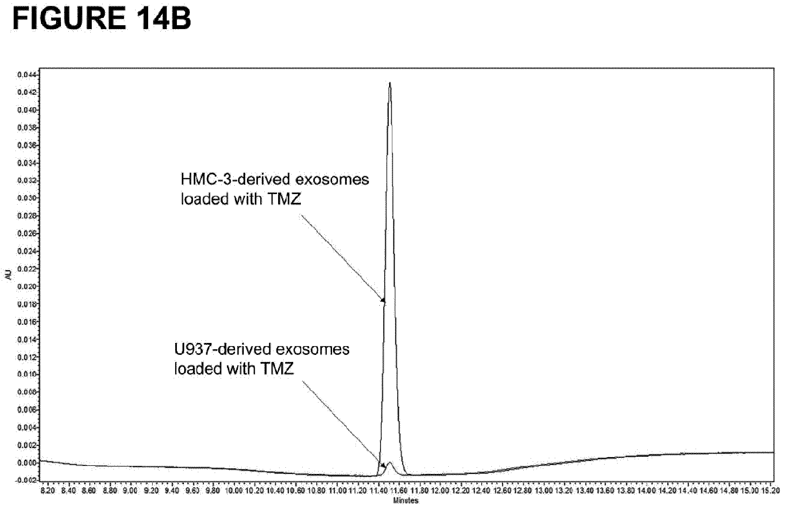

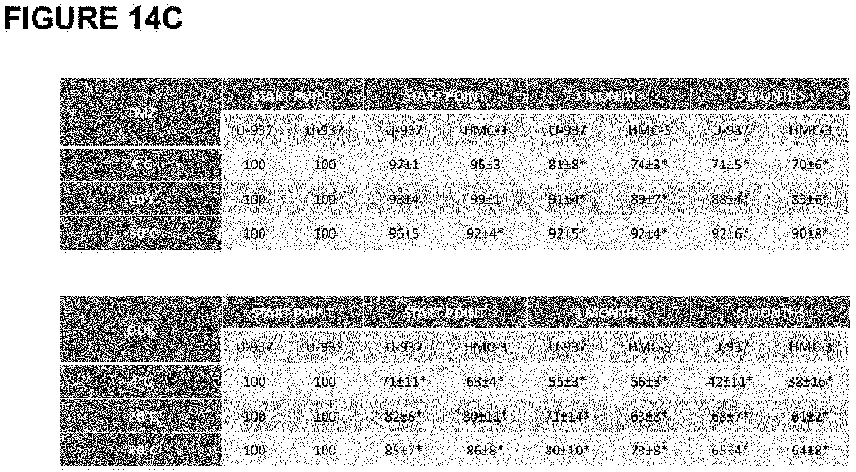

FIG. 14A shows chromatograms and concentration-related fluorescence for doxorubicin (DOX) loaded in EVs derived from HMC-3 microglia and U937 macrophages. FIG. 14B shows chromatograms for temozolomide (TMZ) loaded in EVs derived from HMC-3 microglia and U937 macrophages. FIG. 14C shows the percentage of TMZ and DOX amount per total EV protein unit in EV samples after storage compared with the start point. * means statistically significant decrease compared to the start point.

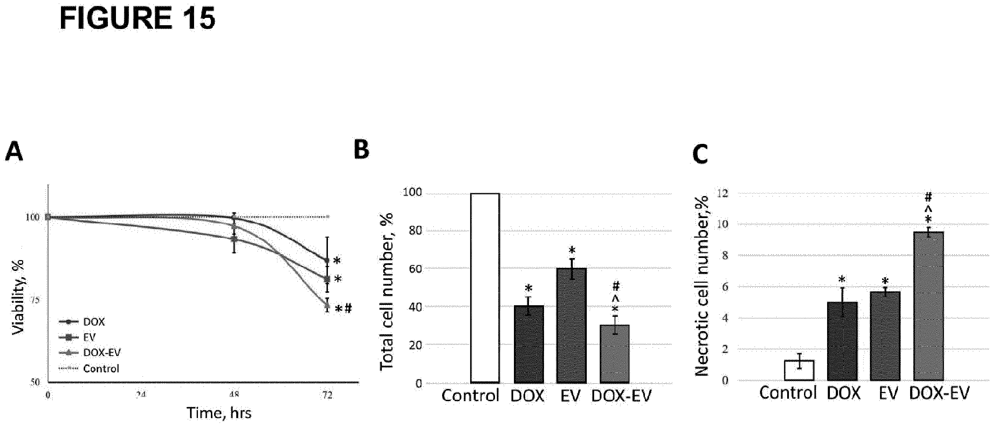

FIG. 15A shows the effect of EVs derived from HMC-3 microglia, free DOX and EV-loaded DOX on the viability of 2D-cultured glioblastoma cells assessed by a metabolic PrestoBlue assay. FIG. 15B shows the effect of EVs derived from HMC-3 microglia, free DOX and EV-loaded DOX on the total cell number in 2D glioblastoma cell cultures after 72 hours. FIG. 15C shows the effect of EVs derived from HMC-3 microglia, free DOX and EV-loaded DOX on the necrotic cell number in 2D glioblastoma cell cultures after 72 hours. The symbol * is for statistically significant difference compared to the Control, ^ - to the DOX, # - to the EV.

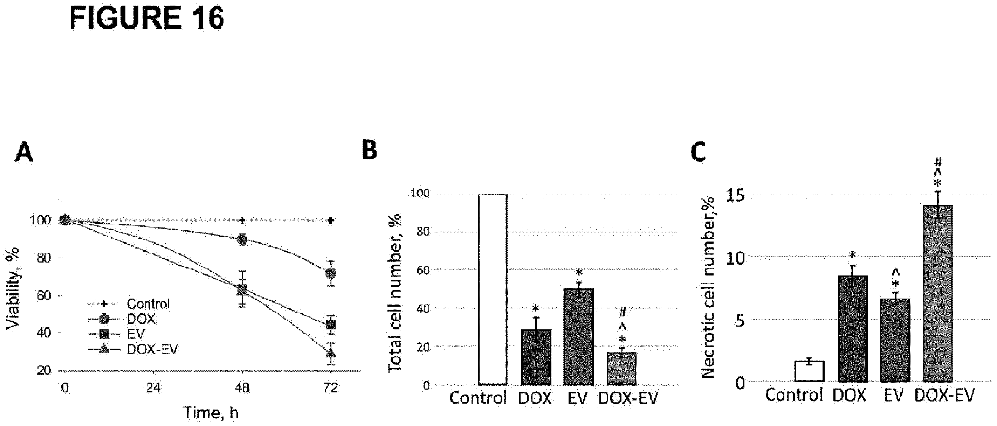

FIG. 16A shows the effect of EVs derived from U937 macrophages, free DOX and EV-loaded DOX on the viability of 2D-cultured glioblastoma cells assessed by a metabolic PrestoBlue assay. FIG. 15B shows the effect of EVs derived from U937 macrophages, free DOX and EV-loaded DOX on the total cell number in 2D glioblastoma cell cultures after 72 hours. FIG. 15C shows the effect of EVs derived from U937 macrophages, free DOX and EV-loaded DOX on the necrotic cell number in 2D glioblastoma cell cultures after 72 hours. The symbol * is for a statistically significant difference compared to the Control, ^ - to the DOX, # - to the EV.

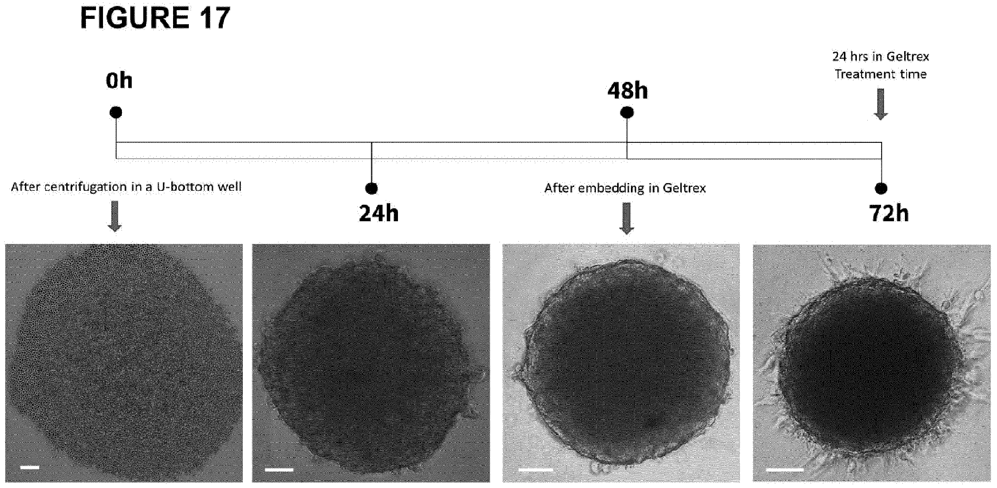

FIG. 17 shows microscope images of 3D glioblastoma spheroid used for tissue-relevant EV efficiency testing at different formation stages.

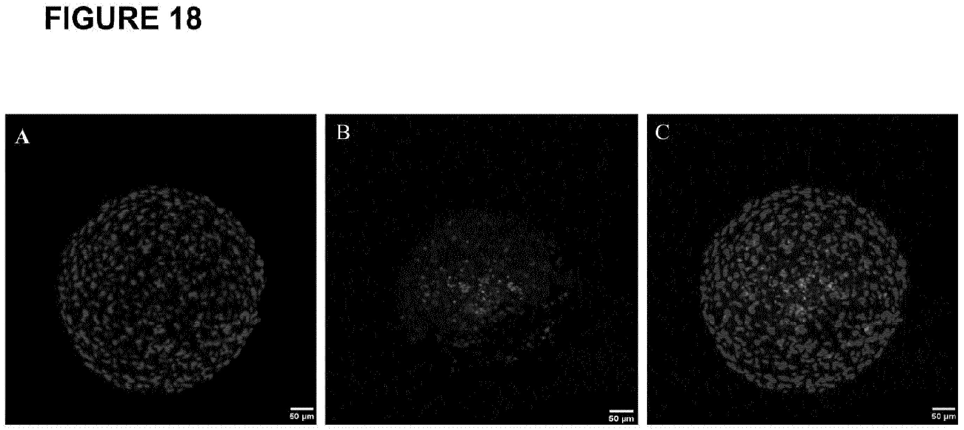

FIG. 18A shows a confocal microscope image of cell nuclei in a developed glioblastoma 3D spheroid. FIG. 18B shows a confocal microscope image of cells that are stained with a Hypoxyprobe^{™} RED 549 for hypoxic core evaluation in a developed glioblastoma 3D spheroid. FIG. 18C shows merged confocal microscope images presented in FIG. 18A and FIG. 18B.

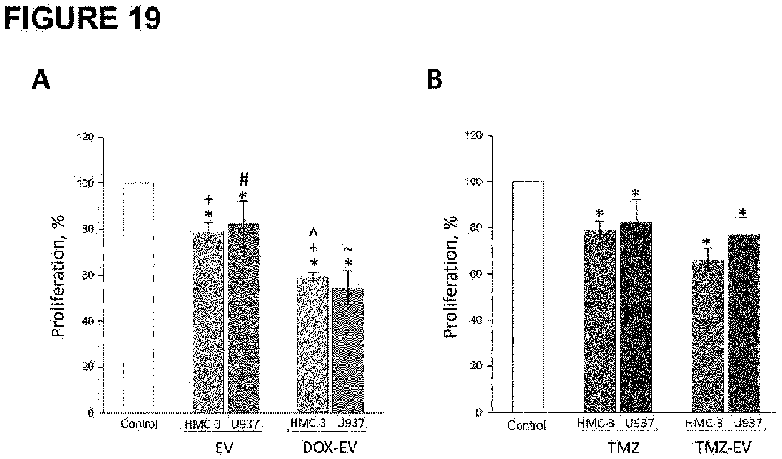

FIG. 19A shows the effect of EVs derived from HMC-3 microglia and U937 macrophages and DOX-loaded EVs derived from HMC-3 microglia and U937 macrophages on glioblastoma cell proliferation in 3D cultures, FIG. 19B shows the effect of EVs derived from HMC-3 microglia and U937 macrophages and TMZ-loaded EVs derived from HMC-3 microglia and U937 macrophages on glioblastoma cell proliferation in 3D cultures. Symbol * defines statistically significant difference compared to the control, # - compared to DOX 0.5 µg/mL, + - compared to DOX 2 µg/mL, ^ - compared with HMC-3 EV, ~ - compared to U937 EV.

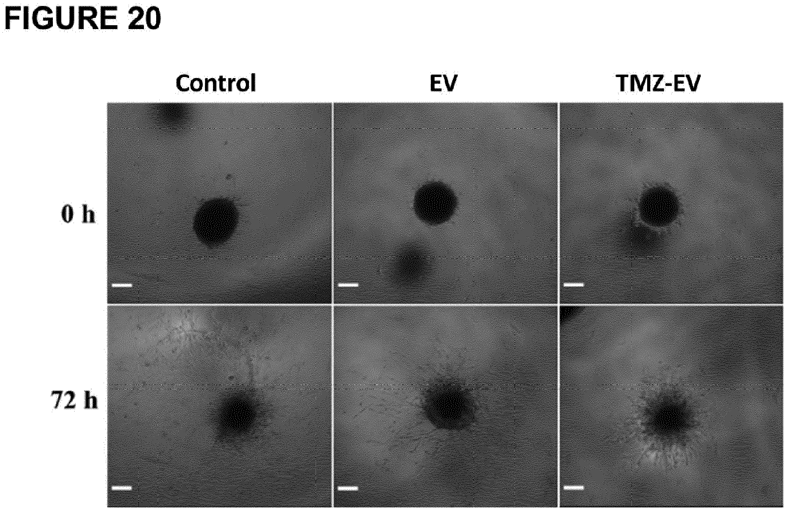

FIG. 20 shows microscope images of Geltrex-encapsulated glioblastoma spheroids treated before and after 72 h treatment with U937 derived EVs, and U937 derived EVs loaded with TMZ,

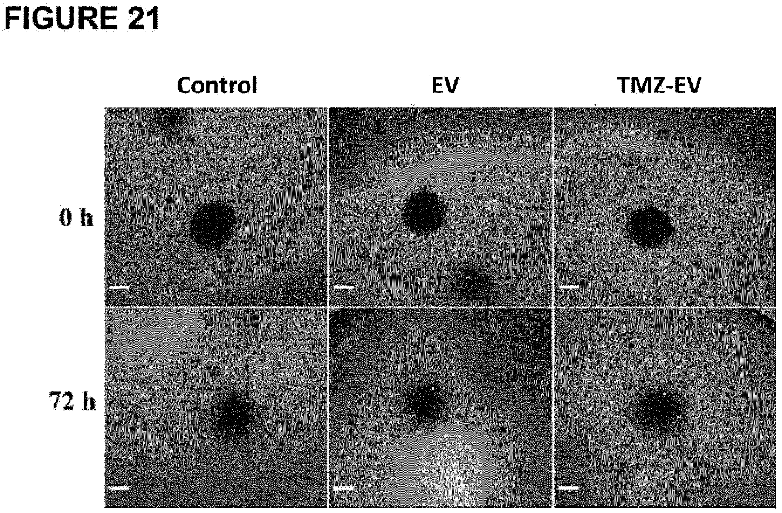

FIG. 21 shows microscope images of Geltrex-encapsulated glioblastoma spheroids treated before and after 72 h treatment with HMC-3 derived EVs, and HMC-3 derived EVs loaded with TMZ.

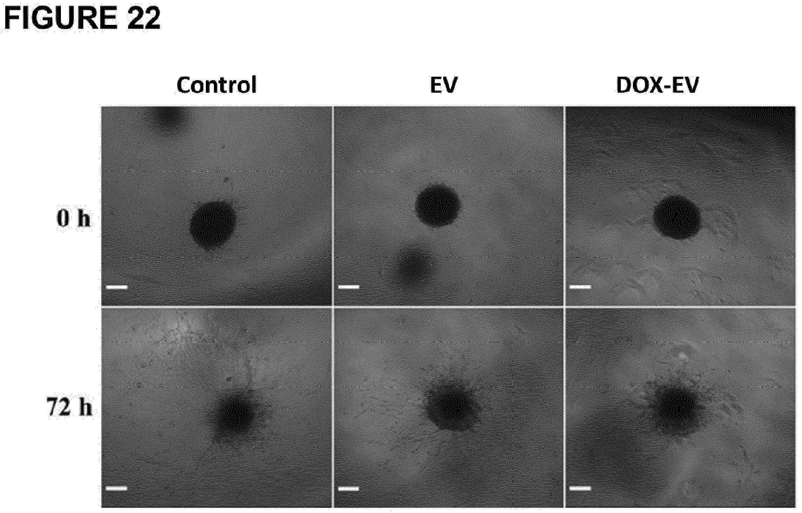

FIG. 22 shows microscope images of Geltrex-encapsulated glioblastoma spheroids treated before and after 72 h treatment with U937 derived EVs, and U937 derived EVs loaded with DOX.

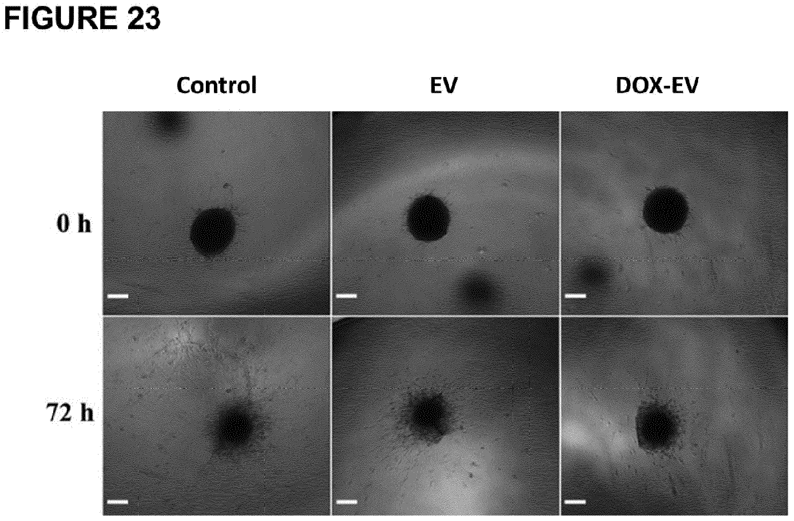

FIG. 23 shows microscope images of Geltrex-encapsulated glioblastoma spheroids treated before and after 72 h treatment with HMC-3 derived and HMC-3 derived EVs loaded with DOX.

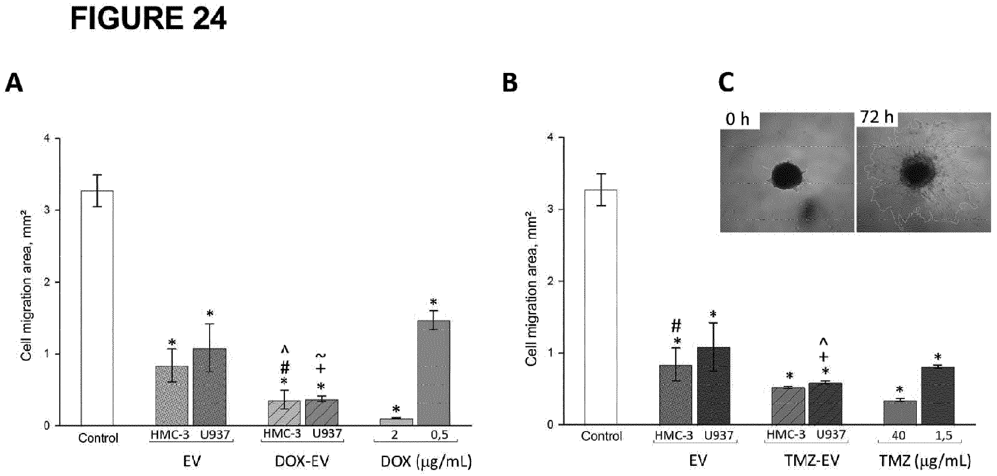

FIG. 24A shows the effect of EVs derived from HMC-3 microglia and U937 macrophages and DOX-loaded EVs derived from HMC-3 microglia and U937 macrophages on glioblastoma cell migration in 3D cultures. FIG. 19B shows the effect of EVs derived from HMC-3 microglia and U937 macrophages and TMZ-loaded EVs derived from HMC-3 microglia and U937 macrophages on glioblastoma cell migration in 3D cultures. FIG. 24C shows an example explaining how the cell migration area the images is defined by ImageJ software. Symbol * defines statistically significant difference compared to the control, # - compared to DOX 0.5 µg/mL, + - compared to DOX 2 µ/mL, ^ - compared with HMC-3 EV, ~ - compared to U937 EV.

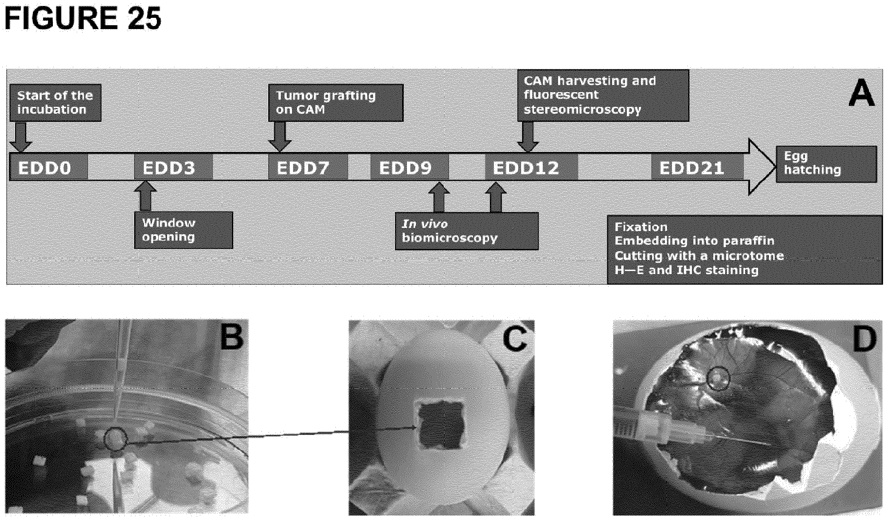

FIG. 25 shows an experimental scheme for tumour (circled) grafting on chick embryo chorioallantoic membrane (CAM).

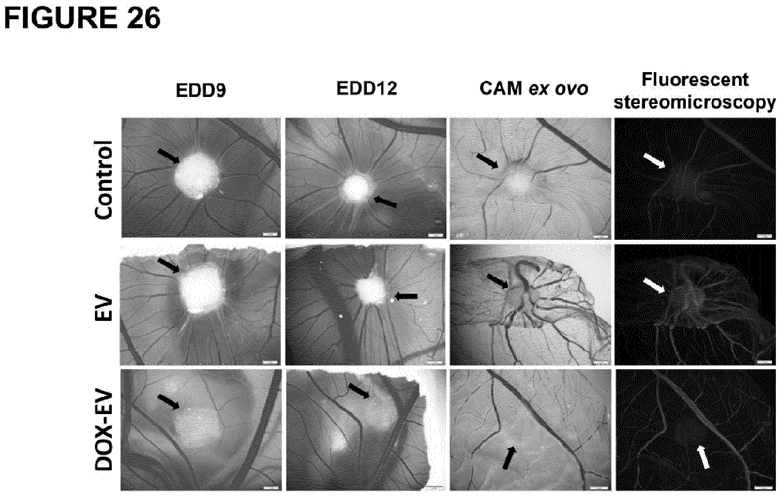

FIG. 26 shows tumour neoangiogenesis on CAM from embryo development day (EDD)9 to EDD 12, CAM *ex ovo,* fluorescent stereomicroscope (scale bar - 1 mm). A thick black or white arrow indicates the tumour.

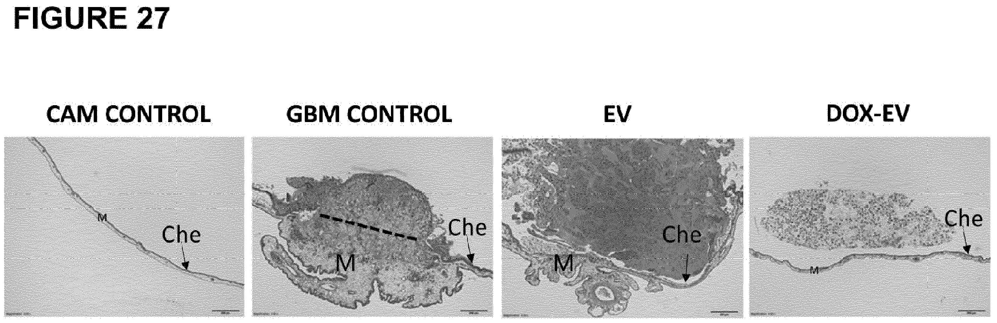

FIG. 27 shows histological staining images representing the effect of macrophage U937-derived EVs and U937-derived EVs loaded with DOX on tumour invasion into CAM. Dotted line - the destruction of chorionic epithelium. ChE - chorionic epithelium, M - mesenchyme. Scale bar - 200 µm.

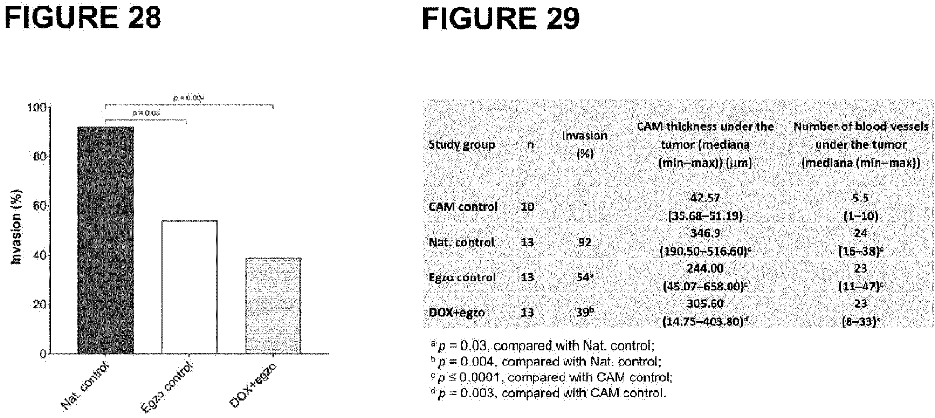

FIG. 28 shows the quantitative evaluation of the effect of macrophage U937-derived EVs and U937-derived EVs loaded with DOX on tumour invasion into CAM frequency.
FIG. 29 shows the effect of macrophage U937-derived EVs and U937-derived EVs loaded with DOX on histomorphometric parameters of HRO636 tumours.

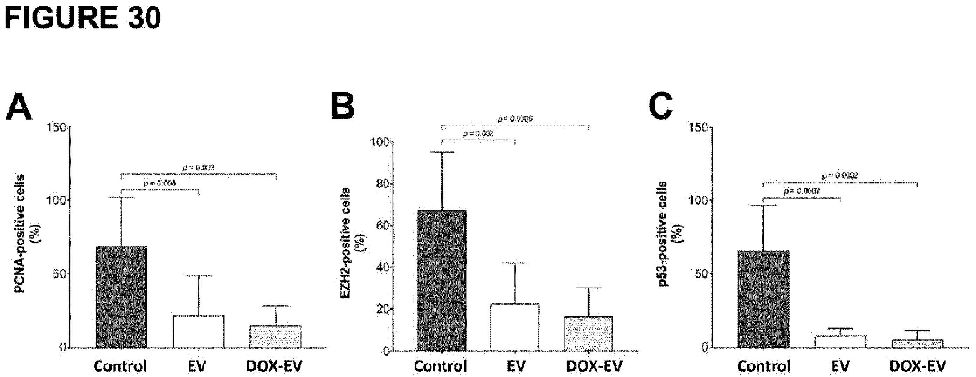

FIG. 30 shows the effect of macrophage U937-derived EVs and U937-derived EVs loaded with DOX on the number of PCNA-, EZH2- and p53 protein-positive cells in the tumours.

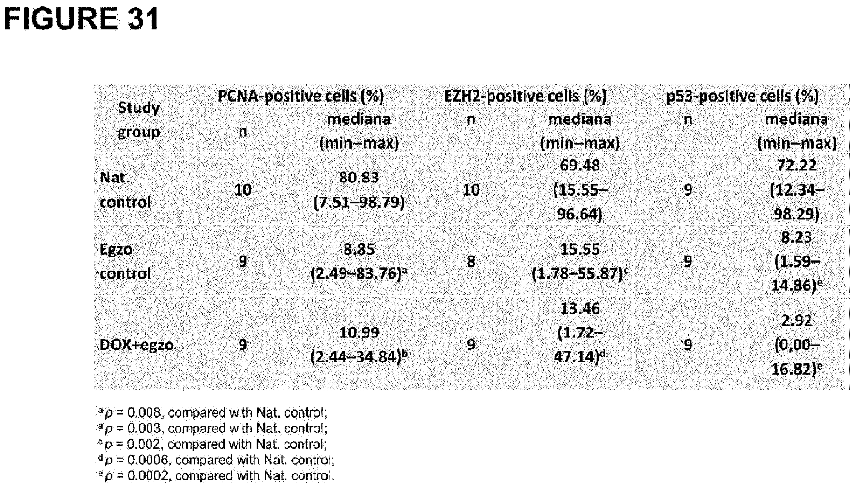

FIG. 31 shows the effect of macrophage U937-derived EVs and U937-derived EVs loaded with DOX on the distribution of PCNA-, EZH2- and p53 protein-positive cells in the tumour samples.

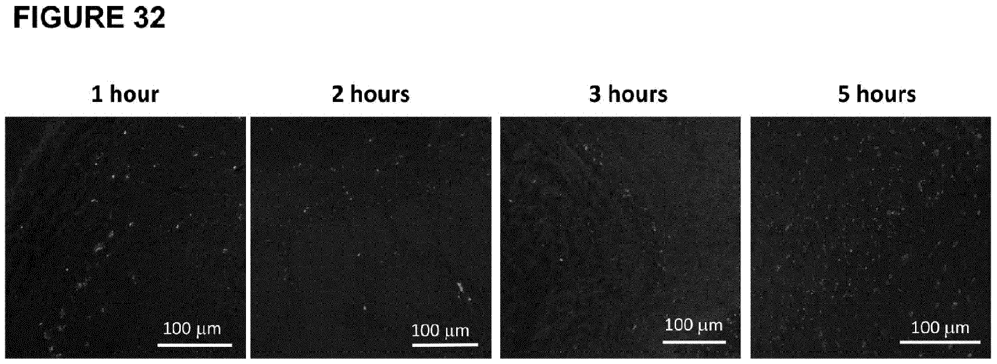

FIG. 32 shows the fluorescence microscope images indicating the presence of macrophage U937-derived EVs with fluorescent cargo in cryoslices of mice brains after different times from intranasal EV delivery.

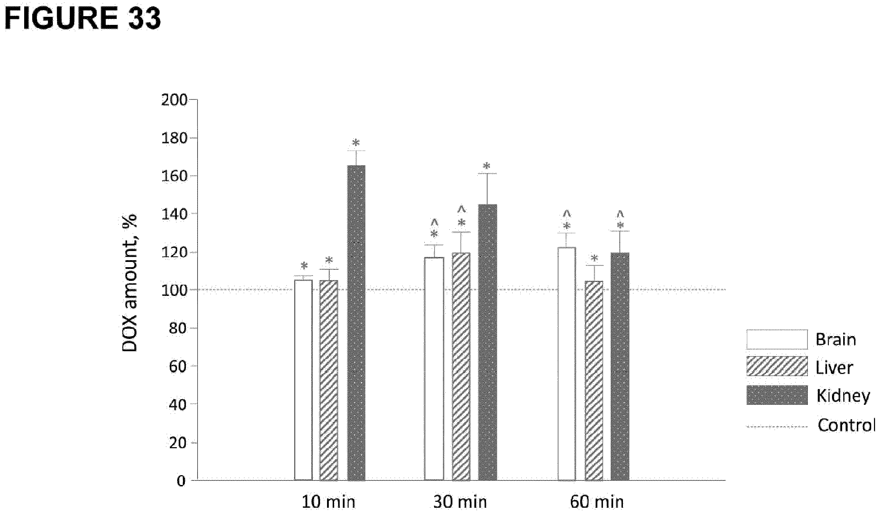

FIG. 33 shows the increase in DOX-dependent fluorescence in mice brain, liver and kidney after intranasal delivery of U937-derived EVs loaded with DOX. The symbol * indicates a significant difference compared with the Control, ^ - with the same organ sample after the shorter time period.

### DESCRIPTION

Macrophages and microglia are cells with multiple phenotypes. They are commonly characterised by the ability to be activated towards inflammatory (M1) or antiinflammatory (M2 phenotype). The tumour environment stimulates the M2 phenotype, thus, the cancer cells are not recognised and removed. Next to the main focus of the immunotherapy to stimulate natural immunity to fight cancer, there is some evidence that macrophage and microglial cell EVs when released from M1 phenotype cells, can directly suppress tumour cell activity.

Microglia make 10-15% of glial cells²⁵. Still, unlike other CNS cells, it develops from the mesoderm of the embryonic yolk sac and circulates in the brain tissue before the blood-brain barrier is fully formed. In the early stages of brain development, microglia are characterised by an amoeboid form that, upon complete formation of the CNS, changes and acquires a high branching around a small oval body with a morphological expression²⁶. It is ramified microglia that is responsible for maintaining CNS homeostasis. The morphological form of the branched microglia allows the cell to change the location of the processes without moving the body, thus assessing the state of the intercellular environment. Constantly moving microglial processes respond rapidly to the onset of a pathological factor in brain tissue and promote cell morphological transformation²⁵. In the presence of neuronal damage or dying cells in the CNS, microglia are activated by transitioning to a hyperbranched form. Subsequently, as the degree of branching of the processes decreases, it becomes reactive (and when the stimulus is prolonged, the processes are fully incorporated into the growing cell body, thus acquiring an amoebic phagocytic morphological phenotype^{27,28}. However, if the activating stimulus is not very strong, microglia undergo a structural transformation to a hyperbranched form²⁷.

EVs primarily studied and used for therapeutical applications are exosomes, which are of 30-150 nm size and are made from maturing endosomes by the intraluminal vesicle (ILV) and multivesicular body (MVB) pathway. The ILV required for MVB formation can be made in two different molecular ways: either by the endosomal sorting complex required for transport (ESCRT) is required for ILV formation. It is a complex of approximately 20 proteins divided into 4 smaller subunits (ESCRT-0, -I, -II, -III) responsible for packaging the respective molecules into ILV. ESCRT-0 is able to recognise ubiquitin-labelled proteins and sort them on the surface of the endosomal membrane. Meanwhile, ESCRT-I and ESCRT-II cause deformation of the endosomal membrane by promoting the formation of its bends inside the endosome. Finally, the binding of ESCRT-III to the auxiliary protein Vps4 initiates the separation and occlusion of the resulting membrane folds, thus forming ILV. ILV may also develop independently of ESCRT function. Lipids such as ceramide, cholesterol and phospholipase D2, the cluster of differentiation (CD) 63, CD81, TSPAN8 and the heat shock protein HSP70 are important for this mechanism. Lipids are responsible for the formation of curves in the endosomal membrane and proteins for packaging appropriate molecular combinations inside the vesicles²⁹.

It has been found that approximately 194 different lipids, 1639 reference ribonucleic acids (mRNAs) and 764 micro-ribonucleic acids (miRNAs) and 4563 proteins can be identified in the structural framework of exosomes³⁰. Cholesterol, sphingomyelin, glycosphingolipids, and phosphatidylserine are the most abundant lipids found in the structure of exosomes. Not only are they responsible for maintaining and limiting the structural form of the vesicle from the external environment, but they are also involved in the processes of exosomal biogenesis. Nucleic acids - mRNA, miRNA and long non-coding RNA - are also detected in exosomes. mRNA can transmit information about the amino acid sequence in the structure of a newly synthesised protein, meaning that an exosome into the target cell with mammalian cell-specific mRNA can induce the synthesis of a non-target cell protein. miRNAs can regulate gene expression in the post-transcriptional period by inhibiting or activating the translation of mRNA-encoding protein with complementary or semi-complementary sequences. miRNAs can also induce histone modifications and methylation of DNA promoter regions during transcription³¹.

Most of the structural elements of the exosomes are of proteins inside and in the membrane. Tetraspanins (CD9, CD81, CD63, CD82, CD54, CD11b) are the most critical exosomal membrane proteins that allow their specific identification. Exosomes are also rich in heat shock proteins (HSP60, HSP70, HSP90), which perform the function of chaperones and regulate the cell response to environmental stimuli. In addition to tetraspanins and heat shock proteins, cytoplasmic proteins such as clathrin, Alix, ubiquitin, TSG101, Rab, and annexins are involved in MVB and, subsequently, exosome formation processes, are also found in exosomes. In the framework of the exosome, class 1 and class II molecules involved in immunoregulation in response to antigenic structures are also identified in the major tissue compatibility complex framework. The exosomes are also rich in enzymes (e.g., protein kinase G, ATPase, enolase, etc.), signal transduction, and even viral proteins³⁰.

In contrast to the exosomes, the content, function and biogenesis mechanism of large EVs is much less understood. The outer membrane budding includes the interaction between the membrane Arrestin Domain-Containing Protein-1 (ARRDC1) with the TSG101 late endosomal protein and subsequent translocation of TSG101 from the endosomal compartment to the plasma membrane³². The membrane is bent by this and ends up with vesicle sliedding³³. In addition, membrane budding can be related to the translocation of lipids such as ceramide or phosphatidylserine³⁴⁻³⁶.

Exomeres are small non-membranous nanoparticles (35 nm and less) enriched in proteins including MAT1A, IDH1, GMPPB, UGP2, EXT1, PFKL and glycan processing factors (MAN2A1, HEXB, GANAB), suggesting they might participate in glycosylation-related processes³⁷.

Recently, a migration-dependent mechanism for EV release and cytoplasm sharing with the EVs was described and named migracytosis³⁸. During migration, a cell leaves a new ring-like organelle, called migrasome, deriving from retraction fibres at the rear edge of the migrating cell³⁹. This process is mediated by integrins gathered into puncta on retraction fibres before migrasome formation. Those integris-ECM interactions are necessary to establish the adhesion sites along the retraction fibres, which are the main migrasome producers in the cell⁴⁰. After the breakdown of the retraction fibres, migrasomes are released and taken up by surrounding cells.

Migrasomes are released by many cells, and macrophages and microglia are one of them. However, the role of these EVs in communication to and from these immune cells is only predictive and remains unknown.

### EXAMPLES

### METHODS

*CELL CULTURES FOR EV PRODUCTION.* Human monocytes U-937 (ATCC, CRL1593.2) were maintained in RPMI-1640 medium with GlutaMAX (Thermo Fisher Scientific) supplemented with 10% exosome-free FBS (Thermo Fisher Scientific) and 1000 IU/mL penicillin-streptomycin (Gibco, Thermo Fisher Scientific). U-937 were grown in T150 (30 mL medium) tissue culture flasks at 37°C (incubator New Brunswick Galaxy 170S, Eppendorf) until the settled cells covered 65-75% of the vial bottom. For induction of cell differentiation, the cell medium was changed to Macrophage-SFM medium (Thermo Fisher Scientific) with 200 nM phorbol myristate acetate (PMA, Merck) and the cells were left for 24 hours in a Soft Flask. After this, the medium was replaced with Macrophage-SFM without PMA and kept in the incubator for 48 hours.

Human microglial cells HMC-3 (ATCC, CRL-3304) were cultivated in DMEM with GlutaMAX (Thermo Fisher Scientific) supplemented with 10% exosome-free FBS and 1000 IU/mL penicillin-streptomycin. When the cell density reached 90-95% of the vessel's surface area, they were detached with 0.25% (w/v) trypsin - 0.53 mM EDTA and plated on Soft Flasks (30 mL of 100 000 cells/mL suspension) for the experiment.

For migration-induced EV shedding, Soft Flask T150 Easy Coat flasks (Cell Guidance Systems) of different stiffness (0.1, 0.2, 0.5, 1, 2, 4, 8, 12, 25, 50, and 100 kPa) were coated with RGD peptide (Merck) solution of different concentrations (0.5, 1, 5, 10, 25, 50, 100, 500, and 1000 mM). The solutions were applied in 15 mL volume per flask and allowed to attach for 8 hours prior to cell seeding.

For EV-shedding imaging on RGD-coated soft surfaces, the cells and EVs were stained with CellMask^{™} Green Plasma Membrane Stain (Thermo Fisher Scientific) according to the manufacturer's protocol and visualised after 30 min post staining by a confocal microscope Zeiss Axio Observer LSM 700 (Carl Zeiss Microimaging Inc., Jena, Germany). The example images of migration-induced EVs are presented in FIG.3 A and B. The average EV number per cell counted in 10 microscopic images obtained from 3 separate experiments (20-35 cells per experiment) was calculated for the most stiffness and RGD concentration selection (the results are presented in FIG. C and D). 12 kPa T150 Easy Coat flasks covered with 100 µM RGD were used for further applications.

For a collection of migration-stimulated EVs, the cells and the EVs were detached by 0.25% (w/v) trypsin - 0.53 mM EDTA. Cells and cell debri were removed by centrifugation at 800 × g for 10 min followed by additional centrifugation at 4000 × g for 20 min. The EVs were collected as the pellet by centrifugation at 20 000 × g for 30 min.

For TLR-3 pathway stimulation, U-937-derived macrophages and HMC-3 cells were treated with 10 µg/mL poly(I:C) for 1 hour (FIG.1A, C). For induction of large EV shedding, the stimulation time was increased to 8 hours (FIG. 1B, D). After the treatments, the large EVs were collected by a series of centrifugation. First, the media were centrifuged for 10 min at 800 × g to remove cells and large cell debri. Then, the supernatant was centrifuged at 14000 × g for 30 minutes to spin down large EVs. The EV pellets were resuspended in PBS by pipetting up and down several times. To identify phosphatidylserine exposure which is characteristic of apoptotic bodies, eBioscience^{™} Annexin V from Apoptosis Detection Kits (Invitrogen, Thermo Fisher Scientific). Briefly, the dye was diluted at a ratio 1:50 in PBS and removed by centrifugation in Exosome Spin Columns (MW 3000) after 15 min of incubation. The recovered EVs were visualised by fluorescent microscope Olympus IX71 (Olympus Corporation, Tokyo, Japan). The images (FIG. 2) were taken by a 01-Exi-AQA-R-F-M-14-C camera (QImaging, Surrey, Canada).

For medium and small EV isolation, final supernatants from migration-induced and poly(I:C)-stimulation-induced EVs were further processed in IZON size exclusion chromatography (SEC) columns qEV10/35nm were used according to the manufacturer's protocol. Briefly, after wanning the column to room temperature, it was immobilised in a laminar flow cabinet and washed with a buffer consisting of PBS. Supernatants from large EV isolation were loaded to a column (10 mL per column), and fractions with a volume of 1 mL were collected (50 fractions per column in total). The total protein content was determined by the Bradford assay (Bradford solution from Sigma-Aldrich), measuring the absorption of λ = 595 nm light in a Tecan Infinite 200 PRO (Tecan Trading AG, Männedorf, Switzerland) plate reader in every second fraction collected (FIG. 6). Each fraction was analysed for dynamic light scattering in a Zetasizer (Zetasizer Nano ZS, Malvern Instruments, UK) and pooled according to their size for further concentration by Amicon Ultra-4 filtration tubes with a 10 kDa threshold (Merck) by filtration at 4000 × g for 10 min and 4000 × g for 30 min. Particle distribution images after concentration are presented in FIG. 7C and D.

For further investigation by transmission electron microscopy (TEM), large EVs (migration-induced and poly(I:C)-stimulation-induced EVs, representative particle distribution analysis in FIG. 7B) and concentrated small EVs were collected together into one all-size range EV pool and analysed for particle size distribution (Fig. 7A), size and morphology by transmission electron microscopy (TEM) and biomarkers.

For TEM imaging, the EVs were processed similarly as described in Kulakauskiene et al.¹⁷ First, the samples were triturated for 5 min with a 30G syringe needle and mixed with 4% paraformaldehyde at the ratio of 1 :1. The solution was applied on carbon-coated Formvar copper meshes FCFT200-Cu-50 200 MESH (Sigma-Aldrich, Merck) and fixed in 1.7% glutaraldehyde solution for 5 min, washed twice in ion-free water for 2 min, stained with 2% uranyl acetate for 2 min, and incubated with freshly prepared 2.25% methylcellulose and 2% uranyl acetate in a v/v ratio of 4:1 for 10 min on ice. Prepared meshes were carefully dried on filter paper for 10 to 15 min and visualised using Tecnai BioTwin Spirit G2 (FEI, Eindhoven, Netherlands) on 80 kV voltage. Electron microscope images were taken with a bottom-mounted 16 MP TEM CCD camera Eagle 4K employing TIA (FEI, Eindhoven, Netherlands). The images are presented in FIG. 4 and 5).

For further applications, pooled samples of all collected EV ranges from 8h poly(I:C)-treated macrophages or microglia grown in the 12 kPa T150 Easy Coat flasks covered with 100 µM RGD were used. The samples were comprised of (i) resuspended pellets of large EVs collected from the medium, (ii) resuspended pellets of surface-attached EVs from macrophages or microglia, and (iii) medium and small EVs. The EVs were subjected to 1 min sonication in an ultrasonic bath (Elmasonic P120H, Elma Schmidbauer, Singen, Germany) at a frequency of 37 kHz, power 100 W, and filtered through 0.22 µm PVDF membranes. The obtained EVs were approximately 50 - 150 nm in diameter (FIG. 8A and B). The EVs of such size are suitable for sterile filtration with a minimal sample loss and are faster taken up by other cells compared to larger EVs. For EV marker analysis control from untreated cells, the EVs were collected the same way, but the macrophages and microglial cells were grown on untreated and uncoated T150 cell culture flasks (TPP) and were not stimulated with poly(I:C).

The markers in the above-described EV lysates made with NP40 Cell Lysis Buffer (Thermo Fisher Scientific) were quantified by ELISA kits. For CD9, CD63, CD81, NDST1. MAT1A, GMPPB, CD80, CD86 - from Abbexa, for IDH1, AGO1, APP, VDAC, COXIV - from Abeam, and for MHC-II - from MyBioSource. The EVs were polymer-sedimented by Total Exosome Isolation Reagent (Thermo Fisher Scientific) according to the manufacturer's instruction, and the pellets were treated with the lysis buffer (0.3 mL per pellet from approximately 1mL pooled EV sample solution with EV protein density ranging in 0.5-3.5 mg/mL) for 30 min. The lysates were analysed by ELISA according to the manufacturer's instructions. The optical density was evaluated in a plate reader Tecan Infinite 200 PRO (Tecan Trading AG, Männedorf, Switzerland). The results are presented in FIG. 9.

For doxorubicin (DOX) and temozolomide (TMZ) loaded EVs, U-937-derived macrophages and HMC-3 microglial cells were subjected to 10 mm/mL DOX, or 50 mm/mL TMZ, respectively, from the moment of plating in hydrogel-RGD-coated flasks. All other treatments and EV isolation procedures were performed as described above with an additional step of removal of not incorporated DOX and TMZ by centrifugation in Exosome Spin Columns (MW 3000).

DOX and TMZ in final EV samples were detected by HPLC. The EV samples were mixed with acetonitrile (1: 1) for 10 min before chromatographic analysis and subjected to ultrasound (Elmasouic P, frequency of 80 kHz, power of 100 W, at room temperature) to break up the vesicle membrane and disperse the inserted drug in the solvent. Before chromatographic analysis, the samples were filtered through 0.2 µm pore size filters. The analysis was performed by Waters e2695 Alliance system (Waters, Milford, MA, USA) and ACE Super C18 column (250 mm × 4.6 mm, particle size 3 µm). The analysis was performed using gradient elution. For the analysis of exosomal samples with TMZ, the mobile phase consisted of A - 0.1% trifluoroacetic acid and B - 100% methanol (0 min, 10% B; 0-3 min, 20% B; 3 - 6 min, 40 % B; 6 - 10 min, 10% B; 10 - 15 min, 10% B). Exosomes in which DOX was inserted were analysed using a mobile phase consisting of A - 0.1% trifluoroacetic acid and B - 100% acetonitrile (0 min, 15% B; 0 - 8 min, 25% B; 8 -16 min, 40% B, 16 -25 min, 70% B, 25 - 33 min, 100% B, 33 - 35 min, 15% B, 35 - 40 min, 15% B, a methodology adapted on the basis of Michels et al.⁴¹ Mobile phase flow rate was 0.5 mL/min. The injection volume of the samples is 10µL. Column temperature - 35 °C. The results of the analysis were processed with the Empower (Waters) software. TMZ and DOX were identified by their retention times and UV spectra of their standards, with TMZ absorption peak at 330 nm and DOX - at 233 nm. For the quantitative evaluation of DOX and TMZ, calibration curves of the standards were made. Representative chromatograms and the amount of DOX determined by fluorescence are presented in FIG. 14A and B.

The same HPLC method was applied for the stability of DOX and TMZ amounts in EV testing. For loaded drug stability evaluation, the samples were stored for 1, 3 and 6 months at 4°C, -20°C and -80°C. The stability results expressed as a percentage of initial drug amount per EV protein weight unit as averages±SDEV of 3 samples measured in 3 technical repeats are presented in FIG. 14C.

For *in vitro* and *in* vivo tracking, the EVs were loaded with AF555-conjugated oligonucleotides (BLOCK-it Alexa Fluor Red Fluorescent Control, Invitrogen, Thermo Fisher Scientific, Vilnius, Lithuania) by the lipofection (RNAiMAX, Invitrogen, Thermo Fisher Scientific, Lithuania, Vilnius). Briefly, a mixture of 0.2 µM of AF555-oligonucleotide conjugate was mixed with 3 µL of RNAiMAX reagent in 100 µL of the Opti-MEM medium (Gibco, Thermo Fisher Scientific) and incubated for 5 min in room temperature. Then, EV samples (1 mg/mL of total protein) were added to the lipofection mixture and incubated at 37 °C for one hour. After incubation, unincorporated dye and residual micelles were removed by Exosome Spin Columns (Invitrogen, Thermo Fisher Scientific). After the labelling and cleaning procedure, the EVs were concentrated by 100 K Amicons (Merck).

*CELL CULTURES FOR EFFICIENCY TESTING.* HROG-36 GBM line cells (CLS) were maintained in DMEM/F12 medium supplemented with 10% FBS and 1% penicillin-streptomycin solution (10000 IU/mL) at 37°C with 5% CO₂. The culture medium was changed 1-2 times a week.

For 2D GBM cell cultures, HROG-36 cells were seeded in a 96-well plate at a density of 3000 cells in 200 µl of medium per well. The plate was incubated at 37°C, 5% CO₂ in a humidified environment for 24 hours, allowing the cells to adhere. The next day, the medium was replaced with a new one supplemented with 0.5, or 5, or 25 µg/mL of EVs isolated from untreated U-937-derived macrophages or HMC-3 microglia, EVs from 1h poly(I:C)-treated macrophages or microglia, EVs from 8h poly(I:C)-treated macrophages or microglia producing large EVs on a soft substrate and 8h poly(I:C)-treated macrophages (EV1, EV2, EV3 and EV4 in FIG. 11, respectively), EV4 at a concentration of 5 µg/mL, free DOX (100 ng/mL), and DOX-loaded EV4 making final DOX concentration the same as in the case of free DOX (100 ng/mL). The treatments were identified as DOX (for free DOX), EV-DOX (for EV4 with loaded DOX), and EV (for EV4) in FIG. 15 and 16. Cells were treated with these preparations for 72 hours by incubating at 37°C with 5% CO₂ and 95% humidity and were assessed for total metabolic activity by PrestoBlue assay (after 48 and 72 hours) and viability by double nuclear fluorescent staining (after 72 hours). The results of total cell metabolic activity are presented in FIG. 11, 15A and 16A, and the viability results are in FIG. 12, FIG. 15B, C and FIG. 16B, C.

For Control of EV effect on non-cancerous primary brain cells, primary cerebellar cell cultures comprising 81±4% granule neurons, 14 ± 3% astrocytes and 6 ± 2% microglial cells, as identified in Balion et al.,⁴² were prepared as described in the mentioned study. The rats were maintained at the Lithuanian University of Health Sciences animal house in agreement with the Guide for the Care and Use of Laboratory Rats. Briefly, cerebella were isolated from euthanised postnatal 5-7-day-old Wistar rats and dissociated in a Versene solution (1:5000; Gibco, Thermo Fisher Scientific) with a series of incubation and trituration with Pasteur pipettes until a single-cell suspension. The isolated cells were seeded in a DMEM medium with Glutamax (Thermo Fisher Scientific) supplemented with 5% horse serum, 5% fetal calf serum, 38 mM glucose, 25 mM KCl, and antibiotic-antimycotic (Anti-Anti, Thermo Fisher Scientific). The cells were plated at a density of 0.25 × 10⁶ cells/cm² in 96-well plates coated with 0.0001% poly-L-lysine and kept at 37 °C in a humidified incubator containing 5% CO₂. The cells were treated with EVs derived from 5 µg/mL migration-induced EVs on a soft RGD-coated substrate and 8h poly(I:C)-treated macrophages for 72 hours (the results are in FIG. 13).

The cells were treated with PrestoBlue Cell Viability Reagent (Thermo Fisher Scientific) for total cell metabolic activity evaluation. The procedure was performed according to the protocol provided by the manufacturer. First, 10 µL of PrestoBlue^{™} reagent was added to each well of a 96-well plate containing 90 µL of cell culture medium and incubated for 40 min at 37°C in a cell culture incubator, protected from direct light. After incubation, the fluorescence of resorufin was detected using a multimode plate reader × at excitation and emission wavelengths of 560 and 590 nm, respectively. The results were expressed as means ± standard deviation of the percentage of untreated control for each experiment.

For viability assessment, the cultures were assessed by double nuclear staining with fluorescent dyes Hoechst33342 (6 µg/mL) and propidium iodide (3 µg/mL) for 10 min and visualised by a fluorescent microscope Olympus IX71 (Olympus Corporation, Tokyo, Japan). The images were taken by a 01-Exi-AQA-R-F-M-14-C camera (QImaging, Surrey, Canada). The image analysis was performed by the ImageJ software.

For EV internalisation assessment in 2D GBM cell cultures, AF555-labelled EV solutions in PBS were applied to mixed glial and microglial cultures making a final concentration of 50 µg/mL of total EV protein. The cells were visualised by a fluorescent microscope Zeiss Axio Observer.Z1 (Carl Zeiss, Jena, Germany). The images are presented in FIG. 10.

For 3D GBM cell cultures, U-bottom 96-well plates were coated with a thin layer of 2% agarose prior to cell seeding. The cells were seeded at a density of 10 000 per well, and the plate was centrifuged at 280 × *g* for 7 min. After 48 hours, the spheroids were transferred to the Geltrex matrix (Thermo Fisher Scientific), and the plate was gently agitated to allow the spheroid to sink into the matrix. Afterwards, the wells were supplemented with a 200 µL incubation medium and further incubated at 37°C in 5% CO₂ for 24 hours. The spheroid diameter was monitored under a light microscope on each day of culture (FIG. 17). The average diameter of the spheroids at this stage was 376 ± 20 µm.

Hypoxic core development in GBM 3D culture testing was evaluated by Hypoxyprobe Kit (Hypoxyprobe Inc). The spheroids were embedded in Geltrex and incubated for 24 hours in glass-bottom 35P dishes (Eppendorf). First, the spheroids were treated with 200 µM pimonidazole hydrochloride for 2 h at 37°C. After incubation, residues of the unbound hypoxia marker were removed by washing with PBS. Next, for labelling pimonidazole hydrochloride-thiol group complexes formed in hypoxic cells, the spheroids were incubated with RED 549 MAb1 (20 µg/mL) under protection from direct light for 2 hours at 37°C. After incubation, the excess dye was removed by washing the plate 3 times with PBS. Next, GBM cell nuclei were labelled by incubation with 10 mM Hoechst33342 fluorescent dye for 30 min. The excess dye was removed by washing with PBS. The images were obtained by a confocal microscope Olympus FluoView1000 (Olympus, Corporation, Tokyo, Japan) and processed with ImageJ freeware. A representative image of hypoxic cells in GBM 3D culture is presented in FIG. 18.

For EV efficiency on GBM cell migration in 3D GBM cell cultures, microscopic images of the cultures were taken at the start and at the end (72 hours), and the area covered by cells was calculated. Spheroid imaging was performed under a light microscope Leica Dmi1 (Leica Microsystems GmbH, Wetzlar, Germany), and the resulting images were used to determine cell migration activity. Using the ImageJ software package, the area occupied by migrating spheroid cells was assessed, and the area of the spheroid at the initial time point was subtracted, as indicated in FIG. 24C. Representative images before and after treatment with U-937-derived macrophage and HMC-3 microglial EVs with and without DOX or TMZ and the same amount of free DOX are presented in FIG. 20 - 23, and quantitative assessment results - in FIG. 24.

The effect of the EVs on total GBM metabolic activity in 3D cell cultures was evaluated by PrestoBlue assay, as described for 2D cell cultures, except for the incubation with PrestoBlue reagent time was prolonged to 3 hours for penetration to the spheroid. The results are presented in FIG. 19.

*GLIOBLASTOMA TUMOURS ON CHORIOALLANTOIC MEMBRANES FOR EFFICIENCY TESTING.* A scheme of tumour grafting on the chorioallantoic membrane (CAM) is presented in FIG. 25 A. Fertilised Cobb-500 chicken eggs were purchased from a local hatchery and incubated at 37°C and 60 % relative air humidity. For three consecutive days, eggs were rolled in an incubator once per hour. Automatic rotation was stopped on the 3rd day of embryo development (EDD3). A small hole was drilled in the location of an air sac, and 2 mL of albumin was aspirated in order to detach the CAM from the eggshell membrane. A square window of 1 cm² was opened in the shell of an egg with a drill and covered with sterile transparent tape. The HRO636 cells were grafted onto CAM on EDD7: 1 × 10⁶ cells were resuspended in 10 µL of serum-free media and 10 µL of the type I rat tail collagen. The mixture was pipetted onto a surgical sponge (3 × 3 × 1 mm) as shown in FIG. 25 B and C. A formed tumour (FIG. 25D, circled) was deposited onto the CAM among major blood vessels. The study groups were as follows: CAM control (n = 10), Nat. control (n = 13), EV control (n = 13), DOX-EV (n = 13), The treatments were started together with the cell planting on CAM. The treatment concentrations were the same as for 3D GBM cell cultures. Tumour growth, neoangiogenesis, and its invasion into CAM were assessed through EDD9-12 by biomicroscopy *in vivo.* At EDD12 fluorescent dextran was injected in the CAM blood vessel (as indicated in FIG. 25D), and the specimens were harvested, fixed in a 10 % formalin solution and embedded into paraffin; 3 µm sections were stained with H-E for morphological study. The p53, EZH2, and PCNA expression in HRO636 tumours were determined by immunohistochemistry (n ≥ 8 in each group).

*IN VIVO EV TRACKING AND DOX TRANSFER EFFICIENCY.* Intranasal EV administration in mice was performed as described in Kulakauskiene et al.¹⁷ All experimental procedures were performed on 10-week-old Balb/c mice according to the Law of the Republic of Lithuanian Animal Welfare and Protection (License of the State Food and Veterinary Service for working with laboratory animals No. G2-96). The mice were maintained and handled at the Lithuanian University of Health Sciences animal house in agreement with the ARRIVE guidelines. Before the intranasal administration of fluorescently labelled EVs, each nostril was treated with 100U hyaluronidase dissolved in 5 µL PBS to disrupt the mucosal barrier. After 30 min, 20 µL of EV solution containing 50 µg of total protein was introduced to each nostril (25 µg per mouse). The solution was administered gradually in 5 µE portions followed by a 5 min interval and alternating the nostrils. After 1, 2, 3, and 5 h, the mice were sacrificed, and the brains were further processed for immunohistochemical analysis.

The brains were washed in PBS and embedded in 4% paraformaldehyde solution for 30 min. Afterwards, the brains were kept in 25% sucrose for 24 h at 4 °C. Then, the tissue was frozen in liquid nitrogen, and serial coronal sections (20 µm thick) containing the were cut at -23 °C using a cryostat (HM 560 Microm, Walldorf, Germany). The sections were mounted on glass slides (Plus, Menzel Glaser, Thermo Fisher Scientific, Vilnius, Lithuania) and allowed for complete dehydration in the cryostat chamber for 10 min. Next, the slides were washed with PBS solution (3 × 10 min) and stained with 0.5 mM 4',6-diamidino-2-phenylindole (DAPI) for 5 min at room temperature for visualisation of the nuclei. The images were taken by a laser scanning confocal microscope Zeiss Axio Observer LSM 700 (Cart Zeiss Microimaging Inc., Jena, Germany). The images are shown in FIG. 32.

For DOX in vivo delivery efficiency, DOX-loaded EVs from the U937-derived macrophages were administered intranasally to male BALB/c mice, similar to EV tracking experiments. The concentration of prepared EV samples per mouse was 10 µg of exosome protein per mouse, making a total volume of 30 µl. The total dose of DOX delivered to one mouse was 10 µg making 0.4 mg per kg of the body weight. Mice were anesthetised 10, 30 and 60 minutes after dosing, the chest cavity was opened, and the beating heart was exposed. The mice were transcardially perfused through the left ventricle (via right atrium incision) with 10 µA of room temperature PBS for 4-5 min to remove blood and EVs that could potentially enter the circulation. After perfusion, the brain, kidneys, and liver were removed, weighed and homogenised in PBS, 2 mL per g of tissue and centrifuge for 20 min at 500 × g. DOX fluorescence in the tissue supernatants was evaluated in a plate reader Tecan Infinite 200 PRO (Tecan Trading AG, Männedorf, Switzerland) using an excitation wavelength of 485 nm and an emission of 595 nm, The results were expressed as an increase in DOX-related fluorescence compared with the untreated tissue control (presented in FIG. 33). *STATISTICAL ANALYSIS.* Statistical analysis was performed using Sigma Plot vl3 software (Systat Software Inc., Berkshire, United Kingdom). The results are presented as means of not less than 3 biological repeats with standard deviation. Statistical comparisons of the two groups were performed using Student's t-test. Multiple groups were compared using and one-way analysis of variance (ONE WAY ANOVA) with the Bonferroni statistical criterion. Differences between means were considered statistically significant at p < 0.05.

For CAM *in vivo* efficiency experiments, the statistical analysis was performed using IBM SPSS v23.0. The frequency of invasion into CAM is expressed as a percentage (%), and the Chi-square test was used to compare tumour invasion into CAM frequency between the study groups. The Shapiro-Wilk test was used to verify the normality assumption. Data of p53-, PCNA- and EZH2-positive stained cells, the number of blood vessels, and the CAM thickness are expressed as median and range (minimum and maximum values). The difference between the two independent groups was evaluated using the nonparametric Mann-Whitney U test. Differences at the value of p < 0.05 were considered significant.

### RESULTS

The cancer environment is characterised by M2 macrophages that cannot recognise and remove malignant cells, The strategies employing macrophage stimulation toward the M1 phenotype by inflammatory modulators poly(I:C), LPS and MS-macrophage-derived EVs demonstrate promising results, and some of the approaches are already being tested in clinical trials^{18,19,21,22,43,44}. Microglia are macrophage analogues in the brain and are similarly affected by cancer cells, but possible exploitation of the cells for brain tumour treatments is less studied. Next to the ability to instruct tumour-associated macrophages and microglia converting towards anticancer phenotype M1, there is some evidence that macrophage- and microglia-derived EVs can directly suppress cancer cells^{13,45}. In this research, we have tested whether EVs isolated from TLR-3 receptor agonist poly(I:C)-stimulated macrophages and microglia can affect GBM cells in 2D and 3D *in vitro* models and in the GBM tumour grafted on CAM We have observed that 4-hour poly(I:C) treatment stimulated the shedding of large EVs from both macrophages and microglia (FIG. 1). The EVs were intensively stained for Annexin V, indicating phosphatidylserine exposure characteristic for apoptotic bodies (FIG. 2). Total cell numbers in U-937-derived macrophage cell cultures were decreased by 15±2% 24 hours after the poly(I:C) treatment (data not shown), confirming a possible increase in apoptotic cell death. However, this was not the case for human microglial cultures, where cell numbers significantly increased by 22±7% over the same period (data not shown). One of the possible explanations for this could be compensation for cell death by microglial proliferation, which does not occur in M1 macrophage culture. However, macrophage and microglial activations are characterised by a membrane blebbing that is not linked to apoptosis;^{46,47} thus, the exact identification and biological meaning of the EVs must be determined by further investigation. Nevertheless, the treatment generated a substantial amount of large EVs, the efficiency of which on tumour cells was unknown as mainly studied EVs are small and medium-size such as exosomes.

Another group of large EVs little is known about are migrasomes, or EVs shedded by fast-moving cells⁴⁸. The substrate favourable for migrasome shedding was described as a stiff fibronectin coating of plastic labware. However, our experience with primary microglial cells migrating faster on soft collagen mimicking hydrogels compared to the rigid plastic surfaces⁴⁹ suggested there might be a specific stiffness and adhesion range more favourable for migration and EV-shedding by the cells. To find out this range, we have performed a series of experiments assessing microglial and macrophage migration on commercial hydrogel matrices characterised by different stiffness and coated with varying concentrations of fibronectin adhesion peptide RGD. The results indicated that macrophages and microglia were moving and leaving surface-attached EVs on the matrices (FIG. 3A, B), and the optimal ranges for the most efficient EV shedding were between 8 and 25 kPa stiffness and between 100 and 500 µM RGD concentration for surface coating (FIG. 3C, D).

Investigation of the morphology of isolated EVs by TEM revealed the presence of EVs from above 3 µm (FIG. 4D) down to about 20 nm in diameter (FIG. 5E, F). Some large EVs contained multivesicular or multi compartmental structures characteristic of migrasomes and apoptotic bodies (FIG. 4B, C). In their turn, small EVs were characterised by a clear round shape and double lipid bilayer (FIG. 5B-D). An example of a small EV with surface concavity indicates the EVs have internal luminal space (FIG. 5A).

Fractionation of total EV samples indicated protein presence in nearly every fraction of potentially possible EV size (FIG. 6). Size versus number distribution analysis by dynamic light scattering revealed the presence of all EV sizes in the pooled EV samples, starting from about 5 nm and rising up to 10 mm. Although the isolation procedures were not optimised for the isolation of the smallest and the largest detected size EVs, their presence indicates that the production source is available to generate such EVs, and by adapting the procedures to smaller or larger targets, EV yield is likely to increase. Low EV yield is one of the main challenges in designing EV-based treatment strategies. Usually, the EV samples are limited to small and medium-size EVs such as exosomes, and there are some practical reasons for this. Most of the cell-derived samples are sterile-filtered through 0,22 µm membranes to ensure the safety of further applications. However, such filtration eliminates most of the larger EVs. In addition, small EVs, such as exosomes, are optimal for cell internalisation, tissue penetration and biological barrier crossing efficiency. In this study, we collected large EV fractions from macrophages and microglia and broke the EVs into smaller ones by sonication. This resulted in an increased amount of small exosome-sized EVs in the samples (FIG. 8A, B). The total EV protein in the samples isolated from the same amount of cell culture medium increased nearly five times after the addition of 8h-poly(I:C) stimulation-induced EVs and further increased by 78% after the addition of migration-induced EVs (data not shown). Thus, the described assay helps to generate large amounts of EVs potentially applicable as therapeutical substances or drug carriers.

The biomarker analysis of the samples revealed the presence of endosomal components tetraspanins CD9, CD63 and CD81, suggesting exosomes⁵⁰, exomere markers MAT1A (S-adenosylmethionine synthase isoform type-1), IDH1 (isocitrate dehydrogenase 1), mannose 1 phosphate guanyltransferase-b (GMPPB), and AGO1 (argonaute-1), and APP (amyloid precursor protein) according to Zhang et al.⁵¹, migrasome marker NDST1 (bifunctional heparan sulfate N-deacetylase/N-sulfotransferase 1)⁵², mitochondrial markers VDAC (voltage-dependent anion channel, or porin from the outer mitochondrial membrane) and COXIV (the subunit IV of mitochondrial respiratory chain complex IV cytochrome c oxidase), and M1 activation phenotype markers CD80, CD86, and MHC-II (FIG. 9A). Mitochondrial components are common in migrasomes⁵³, and could also be present in apoptotic bodies as they comprise relatively large cell parts. The exosome and exomere markers were also found in the EV samples isolated from poly(I:C) untreated cells grown on plastic labware. The tetraspanins CD9, CD63 and CD81 were even in significantly higher quantities; this can be explained by the much higher small/large EV ratio in these samples (FIG. 9B). However, the concentration of most of the exomere markers (MAT1A, IDH1, GMPPB) was lower in the untreated controls. The exception was AGO1 which was found at about twice higher amounts. In addition, the EVs from untreated cells did not have detectable amounts of migrasome marker NDST1 and M1 phenotype markers CD80, CD86, and MHC-II. Summarising, the EVs from 8h poly(I:C)-treated macrophages or microglia grown on surface of 12 kPa stiffness coated by RGD are different from the EVs from the untreated cells grown on plastic because they contain not only markers of exomeres and exosomes, but also those of migrasomes, apoptotic bodies, mitochondria and M1 macrophage/microglia activation.

After 2 hour treatment, the EVs from macrophages and microglia were internalised by cultured GBM cells GROG36 (FIG. 10). Moreover, in addition to a significant increase in EV yield, the EVs obtained from large EV fractions resulted in substantial improvement in the anticancer activity of the preparations. As revealed in FIG. 11, the total metabolic activity of HROG36 cells was significantly increased by the addition of each next EV fraction: EVs from 1h poly(I:C)-treated macrophages or microglia (EV2), EVs induced by migration on soft RGD-coated substrate-induced (EV3), and EVs from 8h poly(I:C)-treated macrophages or microglia (EV4). In addition, the effect of the EVs was directly dependent on the concentration.

Double nuclear viability staining confirmed the metabolic activity results indicating an increased amount of necrotic nuclei together with the addition of each fraction (FIG. 12). Interestingly, the effect was not so much revealed in the primary neuronal-glial cerebellar cell cultures; the visible amount of necrotic nuclei in primary brain cell samples was significantly smaller compared to the same treated (with EV4) HROG36 samples (compare FIG. 12D and FIG. 13D).

Next, in the study, we assessed the EVs' capacity to be loaded with anticancer drugs DOX and TMZ by applying the drugs to the EV-producing cells. We and others have previously shown that the EV-producing cells can incorporate the material from the medium into the EVs^{1,17}. We have assumed that the cargo packed into EVs by cells would remain more stable than introduced by EV structure disrupting methods such as electroporation, sonication or freeze-thawing. To test this hypothesis, we have administered DOX and TMZ to the cultured EV-producing macrophages and microglia, determined the drug presence in the EVs by HPLC and fluorescence measurements (for DOX) and evaluated the stability of the drug over time under different temperature conditions. Interestingly, microglial EVs had significantly higher drug loading capacity compared to macrophage EVs (FIG. 14A and B). Stability testing revealed EVs with TMZ were more stable compared to the EVs with DOX (FIG. 15). This, in part, can be explained by the higher sensitivity of DOX molecules. However, the EVs were relatively stable and, after 6 months, still contained about 90 % of TMZ and over 60 % of DOX when stored at -80°C. For comparison, the drugs loaded into EVs by electroporation had to be consumed within a week because of leakage and degradation (data not shown). Thus, the incorporation of the cargo by introducing it to the medium of EV-producing cells is a good strategy to obtain stable loaded EVs.

DOX-loaded EV efficiency testing in 2D GBM cell cultures revealed that EV-loading significantly increased DOX toxicity both in macrophage and microglia-generated EVs. A decrease in total metabolic activity was accompanied by a decrease in total cell number and an increase in necrosis level (FIG. 16 and 17). The efficiency of DOX-loaded EVs in hypoxic core-containing GBM 3D cultures was not so prominent as in 2D cultures (FIG. 19). The EVs alone were still efficient, but the decrease in viability was about 20 % compared to nearly 30 % in 2D environment. The total metabolic activity of 3D GBM spheroids was further significantly decreased by treatment with DOX-loaded EVs, but not with TMZ-loaded EVs; although TMZ-EVs showed a tendency for viability decreasing, the difference was not significant compared to the EV treatment (FIG. 19A, B). This agrees with other studies demonstrating that 3D cancer cell models provide less efficiency primarily due to more tissue-like penetration⁵⁴. However, such models are more reliable and generate closer in vivo results than 2D cell cultures.

Despite lower efficiency on total metabolic activity, the EVs were remarkably efficient in suppressing GBM cell spreading (migration). The EVs without loaded drugs suppressed GBM migration more than twice, and both TMZ and DOX further decreased migration area by another 50 % (FIG. 20 - 23 and 24). DOX and TMZ applied at higher concentrations were more efficient in GBM migration suppression than DOX-EVs and TMZ-EVS, but the loaded EVs were more suppressing at lower concentrations. This might be explained by the more efficient cell internalisation of the EV-loaded drug. On the other hand, at high concentrations, the small drug penetrates cells in the cultures faster than EVs, resulting in higher efficiency.

The closest to in vivo EV testing was performed on tumours grafted on CAMs. Interestingly, in this model, EVs' efficiency was as high as with the drug without the drug.

Neoangiogenesis was induced in Nat. control and EV control groups and was inhibited in DOX-EV group, as seen in FIG. 26. Reduced neoangiogenesis is confirmed by a fluorescence stereomicroscope. Nat. control and EV control tumours are surrounded by newly formed blood vessels, clearly expressed "spoked wheel" pattern is present.

FIG. 28 represents tumour invasion into CAM. In H-E-stained pictures of Nat. Control and EV control tumours destruction of chorionic epithelium is seen (dotted line), tumours have invaded CAM mesenchyme (M). DOX+EV tumour is shown on top of CAM, and the integrity of the chorionic epithelium is intact. An intact CAM of a fertilised chicken egg is considered comparative (CAM control). In EV control and DOX-EV groups, tumour invasion into CAM was lower than in Nat. Control group (respectively, p = 0.03 and p = 0.004) (FIG. 28 and 29). No statistical significance was found in the CAM thickness and the number of blood vessels under the tumour between study groups (FIG. 29). Compared with a CAM control of intact CAM of a fertilised chicken egg, all tumours in study groups increased the thickness of CAM and the number of blood vessels under the tumour (p ≤ 0.003).

FIG. 30 and 31 show tumour proliferation and invasion-linked PCNA, EZH2 and p53 protein expression in the tumours among investigated groups. In EV control and DOX-EV groups, PCNA, EZH2, and p53 protein expressions were lower than in Nat. Control group (p ≤ 0.008).

Overall, macrophage and microglial EVs without DOX demonstrated similar efficiency as the DOX-loaded EVs in *in vivo*-like CAM tumour model study. Such an effect can be due to a relatively low amount of the drug-loaded into the EVs. On the other hand, the direct antitumour efficiency of EVs is also revealed. Such findings indicate the EVs generated this way can be a promising cancer treatment strategy and might even be more efficient in *vivo* than *in vitro.* In addition, the effect of the loaded drug can be better revealed after a more extended treatment period.

EV *in vivo* tracking shows the EVs entering the brain already after one hour (FIG. 32), which is confirmed by other studies showing the EVs (yet from different cell sources) reaching the brain even faster than in 1 hour^{17,55}. Visually, the amount of EVs is the same in the brains after 2 and 3 hours but seems to increase after 5 hours. However, this is just a qualitative evaluation confirming the EV entrance to the brain, but the precise distribution and localisation of the EVs need more consistent investigation.

Assessment of EV-loaded DOX uptake by the brain and other organs revealed no significant increase after 10 min. Still, a much higher increase after 30 min and again a lower level after 60 min (FIG. 33). A high peak of the dox in the kidney after 10 min indicates that this is the main organ of EV-DOX removal. Some increase in DOX fluorescence was also observed in the liver, particularly after 30 min post intranasal introduction. Thus, microglial and macrophage EVs can deliver DOX to the brain. This is an important finding because DOX cannot penetrate the blood-brain barrier on its own; thus, it cannot be directly applied for brain tumour treatment. On the other hand, the EV strategies to deliver DOX to the brain are constantly developed, and a recently published study describes RGD-engineered EVs can efficiently deliver DOX to the brain and inhibit tumour growth⁵⁶.

### NON PATENT REFERENCES

1. Fu, S, Wang, Y, Xia, X. & Zheng, J. C. Exosome engineering: Current progress in cargo loading and targeted delivery. NanoImpact 20, 100261 (2020).
2. Ostrom, Q. T. et al. The epidemiology of glioma in adults: a 'state of the science' review. Neuro. Oncol. 16, 896-913 (2014).
3. Rong, L., Li, N. & Zhang, Z. Emerging therapies for glioblastoma: current state and future directions. J. Exp. Clin. Cancer Res. 2022 411 41, 1-18 (2022).
4. Liu, Y, Shen, Y, Sun, T. & Yang, W. Mechanisms regulating radiosensitivity of glioma stem cells. Neoplasma 64, 655-665 (2017).
5. Paw, I., Carpenter, R. C., Watabe, K., Debinski, W. & Lo, H. W. Mechanisms regulating glioma invasion. Cancer Lett. 362, 1-7 (2015).
6. Di Danieie, A., Antonucci, Y. & Campello, S. Migrasomes, new vescicles as Hansel and Gretel white pebbles? Biol. Direct 17 1-9 (2022).
7. Witwer, K. W. & Théry, C. Extracellular vesicles or exosomes? On primacy, precision, and popularity influencing a choice of nomenclature. https://doi.org/10.1080/20013078.2019.1648167 8, (2019).
8. Malkin, E. Z. & Bratman, S. V. Bioactive DNA from extracellular vesicles and particles. Cell Death Dis. 2020 117 11, 1-13 (2020).
9. Zhang, Q. et al. Supermeres are functional extracellular nanoparticles replete with disease biomarkers and therapeutic targets. Nat. Cell Biol. 2022 2312 23, 1240- 1254 (2021).
10. Kalluri, R. & LeBleu, V. S. The biology, function, and biomedical applications of exosomes. Science (80-.). 367, (2020).
11. Yu, S. & Yu, L. Migrasome biogenesis and functions. FEBS J. (2021) doi:10.1111/FEBS.16183.
12. Liu, J., Wu, F. & Zhou, H. Macrophage-derived exosomes in cancers: Biogenesis, functions and therapeutic applications. Immunol. Lett. 227, 102-108 (2020).
13. Serpe, C et al. Microglia-derived small extracellular vesicles reduce glioma growth by modifying tumor cell metabolism and enhancing glutamate clearance through miR-124, Cells 10, (2021).
14. Kim, M. S. et al. Development of exosome-encapsulated paclitaxel to overcome MDR in cancer cells. Nanomedicine 12, (2016).
15. Banks, W. A. et al. Transport of Extracellular Vesicles across the Blood-Brain Barrier: Brain Pharmacokinetics and Effects of Inflammation. Int. J. Mol. Sci. 21, 1-21 (2020).
16. Zhuang, X. et al. Treatment of Brain Inflammatory Diseases by Delivering Exosome Encapsulated Antiinflammatory Drugs From the Nasal Region to the Brain. Mol. Ther. 19, 1769-1779 (2011).
17. Kulakauskienė, D. et al. Virus Mimetic Poly (I:C)-Primed Airway Exosome-like Particles Enter Brain and Induce Inflammatory Cytokines and Mitochondrial Reactive Oxygen Species in Microglia. Biology (Basel). 10, (2021).
18. Wang, X. et al. Exploration and functionalisation of M1-macrophage extracellular vesicles for effective accumulation in glioblastoma and strong synergistic therapeutic effects. Signal Transduct. Target. Ther. 2022 71 7, 1-16 (2022).
19. Anfray, C. et al. Intratumoral combination therapy with poly(I:C) and resiquimod synergistically triggers tumor-associated macrophages for effective systemic antitumoral immunity. J. Immunother. Cancer 9, e002408 (2021).
20. Wang, P. et al. Exosomes from M1-Polarized Macrophages Enhance Paclitaxel Antitumor Activity by Activating Macrophages-Mediated Inflammation. Theranoslies 9, 1714 (2019).
21. Aminin, D. & Wang, Y. M. Macrophages as a "weapon" in anticancer cellular immunotherapy. Kaohsiung J. Med. Sci. 37, 749-758 (2021).
22. Jorquera-Cordero, C. et al. Extracellular Vesicles from M1-Polarized Macrophages Combined with Hyaluronic Acid and a β-Blocker Potentiate Doxorubicin's Antitumor Activity by Downregulating Tumor-Associated Macrophages in Breast Cancer. Pharmaceutics 14, 1068 (2022).
25. Nayak, D., Roth, T, L. & McGavern, D. B. Microglia development and function. Annu. Rev. Immunol. 32, 367-402 (2014).
26. Xavier, A. L., Menezes, J. R. L., Goldman, S. A. & Nedergaard, M. Fine-tuning the central nervous system: microglial modelling of cells and synapses. Philos. Trans. R. Soc. B Biol, Sci. 369, (2014).
27. Hinwood, M., Morandini, J., Day, T. A. & Walker, F. R. Evidence that microglia mediate the neurobiological effects of chronic psychological stress on the medial prefrontal cortex. Cereb. Cortex 22, 1442-1454 (2012).
28. Taylor, S. E., Morganti-Kossmann, C., Lifshitz, J. & Ziebell, J. M. Rod Microglia: A Morphological Definition. PLoS One 9, e97096 (2014).
29. Kowal, J., Tkach, M. & Théry, C. Biogenesis and secretion of exosomes. Curr. Opin. Cell Biol. 29, 116-125 (2014).
30. Crenshaw, B. J., Sims, B. & Matthews, Q. L. Biological Function of Exosomes as Diagnostic Markers and Therapeutic Delivery Vehicles in Carcinogenesis and Infectious Diseases. Nanomedicines (2018) doi:10.5772/INTECHOPEN.80225.
31. Gusachenko, O. N., Zenkova, M. A. & Vlassov, V. V. Nucleic acids in exosomes: disease markers and intercellular communication molecules. Biochemistry. (Mosc). 78, 1-7 (2013).
32. Tricarico, C., Clancy, J. & D'Souza-Schorey, C. Biology and biogenesis of shed microvesicles. http://dx.doi.org/10.1080/21541248.2016.1215283 8, 220-232 (2016).
33. Nabhan, J. F., Hu, R., Oh, R. S., Cohen, S. N. & Lu, Q. Formation and release of arrestin domain-containing protein 1-mediated microvesicles (ARMMs) at plasma membrane by recruitment of TSG101 protein. Proc. Natl. Acad. Sci. U. S. A. 109, 4146-4151 (2012).
34. Bianco, F. et al. Acid sphingomyelinase activity triggers microparticle release from glial cells. EMBO J. 28, 1043-1054 (2009).
35. Lhermusier, T., Chap, H. & Payrastre, B. Platelet membrane phospholipid asymmetry: from the characterisation of a scramblase activity to the identification of an essential protein mutated in Scott syndrome. J Thromb. Haemost. 9, 1883-1891 (2011).
36. Morel, O., Jesel, L., Freyssinet, J. M. & Toti, F. Cellular mechanisms underlying the formation of circulating microparticles. Arterioscler. Thromb. Vasc. Biol. 31, 15-26 (2011).
37. Zhang, Q. et al. Transfer of Functional Cargo in Exomeres. Cell Rep 27, 940-954.e6 (2019).
38. Huang, Y. et al. Migrasome formation is mediated by assembly of micron-scale tetraspanin macrodomains. Nat. Cell Biol. 2019 218 21, 991-1002 (2019).
39. Ma, L. et al. Discovery of the migrasome, an organelle mediating release of cytoplasmic contents during cell migration. Cell Res. 2015 251 25, 24-38 (2014).
40. Wu, D. et al. Pairing of integrins with ECM proteins determines migrasome formation. Cell Res. 2017 2711 27, 1397-1400 (2017).
41. Michels, L. R. et al. HPLC-UV method for temozolomide determination in complex biological matrices: Application for in vitro, ex vivo and in vivo studies. Biomed. Chromatogr. 33, e4615 (2019).
42. Balion, Z. et al. Cerebellar Cells Self-Assemble into Functional Organoids on Synthetic, Chemically Crosslinked ECM-Mimicking Peptide Hydrogels. Biomol. 2020, Vol. 10, Page 754 10, 754 (2020).
43. Study of PolyIC and PD-1 mAb in Subjects With Unresectable Hepatocellular Carcinoma - Full Text View - ClinicalTrials.gov.https://clinicaltrials.gov/ct2/show/NCT03732547.
44. De Waele, J. et al. A systematic review on poly(I:C) and poly-ICLC in glioblastoma: adjuvants coordinating the unlocking of immunotherapy J. Exp. Clin. Cancer Res 2021 401 40, 1-20 (2021).
45. Jiang, H. et al. M1 macrophage-derived exosomes and their key molecule IncRNA HOTTIP suppress head and neck squamous cell carcinoma progression by upregulating the TLR5/NF-κB pathway. Cell Death Dis. 2022 132 13, 1-15 (2022).
46. Turola, E., Furlan, R., Bianco, F., Matteoli, M. & Verderio, C. Microglial Microvesicle Secretion and Intercellular Signaling. Front. Physiol. 3, (2012).
47. Pfeiffer, Z. A. et al. The nucleotide receptor P2X7 mediates actin reorganisation and membrane blebbing in RAW 264.7 macrophages via p38 MAP kinase and Rho. J. Leukoc. Biol. 75, 1173-1182 (2004).
48. Yu, S. & Yu, L. Migrasome biogenesis and functions. FEBS J. (2021) doi:10.1111/FEBS.16183.
49. Balion, Z. et al. Comparison of Microglial Morphology and Function in Primary Cerebellar Cell Cultures on Collagen and Collagen-Mimetic Hydrogels, Biomedicines 10, (2022).
50. Théry, C. et al. Minimal information for studies of extracellular vesicles 2018 (MISEV2018): a position statement of the International Society for Extracellular Vesicles and update of the MISEV2014 guidelines. J. Extracell. vesicles 7, (2018).
51. Zhang, H. et al. Identification of distinct nanoparticles and subsets of extracellularvesicles by asymmetric- flow field-flow fractionation. Nat. Cell Biol. 20, 332 (2018).
52. Zhao, X. et al. Identification of markers for migrasome detection. Cell Discov. 2019 51 5, 1-4 (2019).
53. Zhang, Y. et al. Migrasomes: From Biogenesis, Release, Uptake, Rupture to Homeostasis and Diseases. Oxid. Med. Cell. Longev. 2022, (2022).
54. Law, A. M. K. et al. Advancements in 3D Cell Culture Systems for Personalizing Anti-Cancer Therapies. Front. Oncol. 11, 5062 (2021).
55. Banks, W. A. et al. Transport of Extracellular Vesicles across the Blood-Brain Barrier: Brain Pharmacokinetics and Effects of Inflammation. Int. J. Mol. Sci. 21, 1-21 (2020).
56. Tian, Y. et al. A doxorubicin delivery platform using engineered natural membrane vesicle exosomes for targeted tumor therapy. Biomaterials 35, 83-2390 (2014).

### PATENT REFERENCES

23. WO2017173034A1 - Biological agent-exosome compositions and uses thereof - Google Patents.
   https://patents.google.com/patent/ WO2017173034A1 /en.
24. US10723782B2 - Exosomes for immuno-oncology and anti-inflammatory therapy - Google Patents.
   https://patents.google.com/patent/ US10723782B2 /en?oq= US10723782.

## Claims

1. A method for producing extracellular vesicles and/or extracellular particles from macrophage or microglial immune cells, wherein the method comprises:
a. stimulating the immune cells by TLR-3 agonist poly(I:C),
b. stimulating the immune cells by surface-attached cell adhesion peptide RGD,
c. stimulating the cultured immune cells by a matrix stiffness, wherein the matrix stiffness is between 8 and 1000 kPa,
d. isolating the extracellular vesicles and/or particles released to the medium of the cultured immune cells using centrifugation and/or size-exclusion chromatography, and/or polymer precipitation, and/or acoustic trapping, and/or tangential filtration,
e. detaching of the matrix-attached extracellular vesicles with Trypsin-EDTA solution and centrifugation, and/or size-exclusion chromatography, and/or polymer precipitation, and/or acoustic trapping, or tangential filtration,
f. combining extracellular vesicles and/or particles obtained in the steps 1d and le,
g. decreasing the size of the large extracellular vesicles in the composition by sonication or extrusion to the range of 10 to 250 nm,
h. confirming the presence of extracellular vesicles exposing phosphatidylserine,
i. confirming the presence of at least three of the proteins selected from the group of CD9, CD63, CD81, Syntenin-1, TSG-101, Alix, Hsp70, Hsp90,
j. confirming the presence of at least two of the proteins selected from the group of MAT1A, IDH1, GMPPB, AGO1, APP,
k. confirming the presence of the protein NDST1,
l. confirming the presence of COXIV and/or VDAC and/or other mitochondrial proteins,
m. confirming the presence of at least three of the proteins selected from the group of CD80, CD86, MHC-II, CD64, CD32, HLA-DR, CD11c.

2. The method of claim 1, wherein the RGD peptide is attached to the surface from 5 to 500 mM RGD peptide solution.

3. The method of claim 1 or 2, further comprising a step of adding an anticancer agent into the composition of extracellular vesicles and/or particles, by introducing said anticancer agent into the immune cell culture medium prior to the step in 1a.

4. The method of claim 3, wherein the anticancer agent is at least one member from the group consisting of: a nucleic acid, an aptamer, a protein, or a phenolic compound.

5. The method of claim 3, wherein the anticancer agent is doxorubicin or temozolomide.

6. A composition for use in the treatment of cancer of human or animal patients, comprising extracellular vesicles obtained by method according to claims 1 to 5.

7. The composition for use of claim 6, wherein the part of the composition contains at least one member from the group consisting of: an apoptotic body, a migrasome, an exosome.

8. The composition for use of claim 6 or 7, wherein the cancer is a brain cancer.

9. The composition for use of claim 8, wherein the cancer is glioblastoma.

10. The composition for use of any one of claims 6 to 9, wherein the administration is selected from the group consisting of oral, topical, intranasal, intravenous, and intratumoral administration.

11. The composition for use of any one of claims 6 to 10, wherein the extracellular vesicles are obtained from autologous cells of the patient.

## Patentansprüche

1. Verfahren zum Herstellen extrazellulärer Vesikel und/oder extrazellulärer Partikel aus Makrophagen- oder Mikrogliaimmunzellen, wobei das Verfahren Folgendes umfasst:
a. Stimulieren der Immunzellen durch den TLR-3-Agonisten Poly(I:C),
b. Stimulieren der Immunzellen durch das an der Oberfläche anhängende Zelladhäsionspeptid RGD,
c. Stimulieren der kultivierten Immunzellen durch eine Matrixsteifigkeit, wobei die Matrixsteifigkeit zwischen 8 und 1000 kPa liegt,
d. Isolieren der extrazellulären Vesikel und/oder Partikel, die in das Medium der kultivierten Immunzellen freigesetzt wurden, unter Verwendung von Zentrifugation und/oder Größenausschlusschromatographie und/oder Fällungspolymerisation und/oder akustischem Einfangen und/oder Tangentialfiltration,
e. Lösen der an der Matrix anhängenden extrazellulären Vesikel mit einer Trypsin-EDTA-Lösung und Zentrifugation und/oder Größenausschlusschromatographie und/oder Fällungspolymerisation und/oder akustischem Einfangen oder Tangentialfiltration,
f. Kombinieren extrazellulärer Vesikel und/oder Partikel, die in den Schritten 1d und 1e erlangt wurden,
g. Verringern der Größe der großen extrazellulären Vesikel in der Zusammensetzung durch Beschallung oder Extrusion in den Bereich von 10 bis 250 nm,
h. Bestätigen des Vorhandenseins von extrazellulären Vesikeln, die Phosphatidylserin exponieren,
i. Bestätigen des Vorhandenseins von mindestens drei der Proteine, die ausgewählt sind aus der Gruppe aus CD9, CD63, CD81, Syntenin-1, TSG-101, Alix, Hsp70, Hsp90,
j. Bestätigen des Vorhandenseins von mindestens zwei der Proteine, die ausgewählt sind aus der Gruppe aus MAT1A, IDH1, GMPPB, AGO1, APP,
k. Bestätigen des Vorhandenseins des Proteins NDST1,
l. Bestätigen des Vorhandenseins von COXIV und/oder VDAC und/oder anderen Mitochondrienproteinen,
m. Bestätigen des Vorhandenseins von mindestens drei der Proteine, die ausgewählt sind aus der Gruppe aus CD80, CD86, MHC-II, CD64, CD32, HLA-DR, CD11c.

2. Verfahren nach Anspruch 1, wobei das RGD-Peptid aus 5 bis 500 mM einer RGD-Peptidlösung an die Oberfläche angehängt wird.

3. Verfahren nach Anspruch 1 oder 2, ferner umfassend einen Schritt des Hinzufügens eines Antikrebswirkstoffs zu der Zusammensetzung aus extrazellulären Vesikeln und/oder Partikeln durch Einführen des Antikrebswirkstoffs in das Immunzellkulturmedium vor dem Schritt in 1a.

4. Verfahren nach Anspruch 3, wobei der Antikrebswirkstoff mindestens ein Mitglied der Gruppe ist, die aus Folgendem besteht: einer Nukleinsäure, einem Aptamer, einem Protein oder einer Phenolverbindung.

5. Verfahren nach Anspruch 3, wobei der Antikrebswirkstoff Doxorubicin oder Temozolomid ist.

6. Zusammensetzung zur Verwendung in der Behandlung von Krebs menschlicher oder tierischer Patienten, umfassend extrazelluläre Vesikel, die durch ein Verfahren nach den Ansprüchen 1 bis 5 erlangt wurden.

7. Zusammensetzung zur Verwendung nach Anspruch 6, wobei der Teil der Zusammensetzung mindestens ein Mitglied aus der Gruppe enthält, die aus Folgendem besteht: einem Apoptosekörper, einem Migrasom, einem Exosom.

8. Zusammensetzung zur Verwendung nach Anspruch 6 oder 7, wobei der Krebs ein Hirnkrebs ist.

9. Zusammensetzung zur Verwendung nach Anspruch 8, wobei der Krebs ein Glioblastom ist.

10. Zusammensetzung zur Verwendung nach einem der Ansprüche 6 bis 9, wobei die Verabreichung ausgewählt ist aus der Gruppe bestehend aus oraler, topischer, intranasaler, intravenöser und intratumoraler Verabreichung.

11. Zusammensetzung zur Verwendung nach einem der Ansprüche 6 bis 10, wobei die extrazellulären Vesikel aus autologen Zellen des Patienten erlangt werden.

## Revendications

1. Procédé pour produire des vésicules extracellulaires et/ou des particules extracellulaires provenant de cellules immunitaires de macrophage ou de microglie, dans lequel le procédé comprend :
a. la stimulation des cellules immunitaires par l'agoniste de TLR-3 poly(I:C),
b. la stimulation des cellules immunitaires par le peptide d'adhérence cellulaire RGD attaché à la surface,
c. la stimulation des cellules immunitaires cultivées par une rigidité de matrice, dans lequel la rigidité de matrice est comprise entre 8 et 1000 kPa,
d. l'isolement des vésicules extracellulaires et/ou des particules libérées dans le milieu des cellules immunitaires cultivées en utilisant une centrifugation et/ou une chromatographie d'exclusion stérique, et/ou une précipitation de polymère, et/ou un piégeage acoustique, et/ou une filtration tangentielle,
e. le détachement des vésicules extracellulaires attachées à la matrice avec une solution de trypsine-EDTA et une centrifugation, et/ou une chromatographie d'exclusion stérique, et/ou une précipitation de polymère, et/ou un piégeage acoustique, ou une filtration tangentielle,
f. la combinaison des vésicules extracellulaires et/ou des particules obtenues dans les étapes 1d et 1e,
g. la diminution de la taille des grandes vésicules extracellulaires dans la composition par sonication ou extrusion dans la plage de 10 à 250 nm,
h. la confirmation de la présence de vésicules extracellulaires exposant de la phosphatidylsérine,
i. la confirmation de la présence d'au moins trois des protéines choisies dans le groupe constitué de CD9, CD63, CD81, Syntenin-1, TSG-101, Alix, Hsp70, Hsp90,
j. la confirmation de la présence d'au moins deux des protéines choisies dans le groupe constitué de MAT1A, IDH1, GMPPB, AGO1, APP,
k. la confirmation de la présence de la protéine NDST1,
l. la confirmation de la présence de COXIV et/ou de VDAC et/ou d'autres protéines mitochondriales,
m. la confirmation de la présence d'au moins trois des protéines choisies dans le groupe constitué de CD80, CD86, MHC-II, CD64, CD32, HLA-DR, CD11c.

2. Procédé selon la revendication 1, dans lequel le peptide RGD est attaché à la surface à partir d'une solution de peptide RGD de 5 à 500 mM.

3. Procédé selon la revendication 1 ou 2, comprenant en outre une étape consistant à ajouter un agent anticancéreux dans la composition de vésicules extracellulaires et/ou de particules, par l'introduction dudit agent anticancéreux dans le milieu de culture de cellules immunitaires avant l'étape 1 a.

4. Procédé selon la revendication 3, dans lequel l'agent anticancéreux est au moins un élément du groupe constitué de : un acide nucléique, un aptamère, une protéine, ou un composé phénolique.

5. Procédé selon la revendication 3, dans lequel l'agent anticancéreux est la doxorubicine ou le témozolomide.

6. Composition pour son utilisation dans le traitement du cancer de patients humains ou animaux, comprenant des vésicules extracellulaires obtenues par un procédé selon les revendications 1 à 5.

7. Composition pour son utilisation selon la revendication 6, dans laquelle une partie de la composition contient au moins un élément du groupe constitué de : un corps apoptotique, un migrasome, un exosome.

8. Composition pour son utilisation selon la revendication 6 ou 7, dans laquelle le cancer est un cancer du cerveau.

9. Composition pour son utilisation selon la revendication 8, dans laquelle le cancer est un glioblastome.

10. Composition pour son utilisation selon l'une quelconque des revendications 6 à 9, dans laquelle l'administration est choisie dans le groupe constitué d'une administration orale, topique, intranasale, intraveineuse, et intratumorale.

11. Composition pour son utilisation selon l'une quelconque des revendications 6 à 10, dans laquelle les vésicules extracellulaires sont obtenues à partir de cellules autologues du patient.
